(19) Europäisches Patentamt
European Patent Office
Office européen des brevets

(11) Veröffentlichungsnummer: **0 068 386**
**B1**

(12) # EUROPÄISCHE PATENTSCHRIFT

(45) Veröffentlichungstag der Patentschrift: **18.12.85**

(51) Int. Cl.⁴: **C 07 D 471/04**, A 61 K 31/44 // (C07D471/04, 235:00, 221:00)

(21) Anmeldenummer: **82105426.9**

(22) Anmeldetag: **21.06.82**

(54) **Substituierte Imidazo(1,5-a)pyridine, Verfahren zu ihrer Herstellung und pharmazeutische Präparate die diese Verbindungen enthalten.**

(30) Priorität: **22.06.81 US 276094**

(43) Veröffentlichungstag der Anmeldung: **05.01.83 Patentblatt 83/1**

(45) Bekanntmachung des Hinweises auf die Patenterteilung: **18.12.85 Patentblatt 85/51**

(84) Benannte Vertragsstaaten: **AT BE CH DE FR IT LI LU NL SE**

(56) Entgegenhaltungen: **JOURNAL OF MEDICINAL CHEMISTRY, Band 16, Nr. 11, 1973 Washington G.J. DURANT et al. "Potential Histamine H2-Receptor Antagonists. 1. Aminoethylimidazo (1,2-a) pyridines and -imidazo (1,5-a) pyridines" Seiten 1272 to 1276**

(73) Patentinhaber: **CIBA-GEIGY AG, Klybeckstrasse 141, CH-4002 Basel (CH)**

(72) Erfinder: **Ford, Neville, Dr., 7146A Dartmouth Avenue, University City Missouri 63130 (US)**

(74) Vertreter: **Zumstein, Fritz sen., Dr. et al, Bräuhausstrasse 4, D-8000 München 2 (DE)**

**EP 0 068 386 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die in der Literatur beschriebenen Imidazo[1,5-a]pyridine sind hauptsächlich nur am Imidazolteil des bicyclischen Ringsystems funktionell substituiert. So sind z.B. 1- und 3-Aminoalkyl substituierte Imidazo[1,5-a]pyridine und ihre Tetrahydroderivate in Journal of Medicinal Chemistry 16, 1272-6 (1973) beschrieben. Für diese Verbindungen sind verschiedene therapeutische Wirkungen beschrieben, auf eine Hemmung der Thromboxan-Synthetase wird jedoch nicht hingewiesen. Es wurde nun überraschend gefunden, dass Imidazo[1,5-a]pyridin-alkansäuren und ihre Derivate eine neue Klasse von ausserordentlich wirksamen und hochspezifischen Thromboxan-Synthetase-Inhibitoren bilden.

Die vorher genannten Vorteile und Eigenschaften tragen dazu bei, dass die Imidazo[1,5-a]pyridinderivate dieser Erfindung, bei Verabreichung, allein oder in Kombination, an Säugern, z.B. für die Behandlung oder Vorbeugung von Krankheiten, welche auf die Hemmung von Thromboxan-Synthetase ansprechen, besonders nützlich sind. Diese Krankheiten umfassen auch kardiovaskulare Störungen wie Thrombose, Atherosklerose, cerebrale ischämische Anfälle, Myokardinfarkt, Angina pectoris und Hypertension; Atmungsstörungen, wie Asthma; Entzündungskrankheiten oder Karzinom, wie Tumormetastasen; und Migräne.

Die vorliegende Erfindung betrifft daher Imidazo[1,5-a]pyridine der allgemeinen Formel (I)

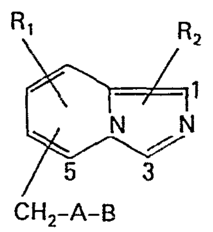

I

oder ihre 5,6,7,8-Tetrahydroderivate, worin jedes der Symbole $R_1$ und $R_2$ Wasserstoff, Halogen oder $C_1$-$C_7$-Alkyl bedeutet, A für ein Alkylen mit 1 bis 12 Kohlenstoffatomen, Alkynylen oder Alkenylen mit 2 bis 12 Kohlenstoffatomen steht, B Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl bedeutet, und ihre Salze, insbesondere ihre therapeutisch verwendbaren Salze, sowie Verfahren zu ihrer Herstellung, pharmazeutische Präparate enthaltend diese Verbindungen, sowie die Verbindungen zur therapeutischen Verwendung.

Bevorzugt sind Verbindungen der allgemeinen Formel (I) in welchen die Gruppe $CH_2$-A-B an die 5-Stellung gebunden ist. Sehr nützlich sind Verbindungen der Formel (I), worin A Alkylen mit 1 bis 12 Kohlenstoffatomen bedeutet.

Besonders bevorzugt sind Verbindungen der allgemeinen Formel (II)

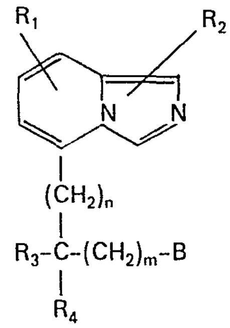

II

oder ihre 5,6,7,8-Tetrahydroderivate, worin jedes der Symbole $R_1$, $R_2$, $R_3$ und $R_4$ Wasserstoff oder $C_1$-$C_4$-Alkyl bedeutet, n eine ganze Zahl von 1 bis 7 ist, m Null oder 1 ist, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze, insbesondere ihre therapeutisch verwendbaren Salze.

Besonders hervorzuheben sind Verbindungen der allgemeinen Formel (II) oder ihre 5,6,7,8-Tetrahydroderivate, worin jedes der Symbole $R_1$, $R_2$, $R_3$ und $R_4$ Wasserstoff, Methyl oder Äthyl bedeutet, $(CH_2)_n$ für Propylen, Butylen, Pentylen oder Hexylen steht, m Null oder 1 ist, B Carboxy, Methoxycarbonyl oder Äthoxycarbonyl, unsubstituiertes Carbamoyl, Monomethyl- oder Monoäthylcarbamoyl, Dimethyl- oder Diäthylcarbamoyl, Cyan oder Hydroxymethyl bedeutet, und ihre Salze, insbesondere ihre therapeutisch verwendbaren Salze.

Überaus nützlich sind Verbindungen der allgemeinen Formel (III)

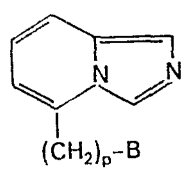

III

oder ihre 5,6,7,8-Tetrahydroderivate, worin p eine ganze Zahl von 3 bis 8 bedeutet, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze, insbesondere ihre therapeutisch verwendbaren Salze.

Besonders wertvoll sind Verbindungen der allgemeinen Formel (IV)

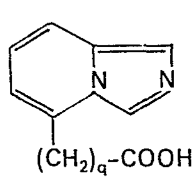

IV

oder ihre 5,6,7,8-Tetrahydroderivate, worin q 4, 5 oder 6 bedeutet, und ihre Salze, insbesondere ihre therapeutisch verwendbaren Salze.

Die allgemeinen Definitionen, welche hier verwendet werden, haben innerhalb des Umfangs

der vorliegenden Erfindung die folgenden Bedeutungen:

Alkylen mit 1 bis 12 Kohlenstoffatomen, kann geradkettig oder verzweigt sein, und bedeutet vorzugsweise Propylen, Butylen, Pentylen oder Hexylen, wobei die genannten Reste unsubstituiert oder durch eine oder mehrere Niederalkylgruppen substituiert sind, mit der Massgabe, dass die Summe der Kohlenstoffatome nicht mehr als 12 ist.

Alkenylen mit 2 bis 12 Kohlenstoffatomen, kann geradkettig oder verzweigt sein, und bedeutet vorzugsweise Propenylen, 1- oder 2-Butenylen, 1- oder 2-Pentenylen, 1-, 2- oder 3-Hexenylen. Die genannten Reste sind unsubstituiert oder durch eine oder mehrere Niederalkylgruppen substituiert, mit der Massgabe, dass die Summe der Kohlenstoffatome 12 nicht übersteigt.

Alkynylen mit 2 bis 12 Kohlenstoffatomen, kann geradkettig oder verzweigt sein, und steht vorzugsweise für Propynylen, 1- oder 2-Butynylen, 1- oder 2-Pentynylen, 1-, 2- oder 3-Hexynylen. Diese Reste sind unsubstituiert oder durch eine oder mehrere Niederalkylgruppen substituiert, wobei die Summe der Kohlenstoffatome 12 nicht übersteigt.

Der Ausdruck «nieder» definiert in den oben oder nachfolgend genannten organischen Resten oder Verbindungen solche mit höchstens 7, vorzugsweise 4, insbesondere 1 oder 2 Kohlenstoffatomen.

Von den $C_1$-$C_7$-Alkylgruppen ist diejenige mit 1–4 Kohlenstoffatomen bevorzugt, z.B. Äthyl, Propyl oder Butyl, insbesondere Methyl.

Eine $C_1$-$C_7$-Alkoxycarbonylgruppe enthält vorzugsweise 1–4 Kohlenstoffatome im Alkoxyteil und bedeutet z.B. Methoxycarbonyl, Propoxycarbonyl oder Isopropoxycarbonyl, insbesondere Äthoxycarbonyl. Eine Mono-($C_1$-$C_7$-alkyl)-carbamoylgruppe hat vorzugsweise 1 bis 4 Kohlenstoffatome in dem Alkylteil und ist z.B. N-Methylcarbamoyl, N-Propyl-carbamoyl oder insbesondere N-Äthylcarbamoyl. Eine Di-($C_1$-$C_7$-alkyl)-carbamoylgruppe enthält vorzugsweise 1 bis 4 Kohlenstoffatome in jeder Niederalkylgruppe und steht z.B. für N,N-Dimethylcarbamoyl, N-Methyl-N--äthylcarbamoyl und insbesondere für N,N-Diäthylcarbamoyl.

Salze sind vorzugsweise therapeutisch verwendbare Salze, z.B. Metall- oder Ammoniumsalze der genannten Verbindungen der Formel I, worin B Carboxy bedeutet, insbesondere Alkalimetall- oder Erdalkalimetallsalze, z.B. Natrium-, Kalium-, Magnesium- oder Calciumsalze; in erster Linie leicht kristallisierende Ammoniumsalze. Diese werden abgeleitet von Ammoniak oder organischen Aminen, z.B. Mono-, Di- oder Trinieder-(alkyl, cycloalkyl oder hydroxyalkyl)-aminen, Niederalkylendiaminen oder (Hydroxyniederalkyl oder Aryl-niederalkyl)-niederalkylammonium Basen, z.B. Methylamin, Diäthylamin, Triäthylamin, Dicyclohexylamin, Triäthanolamin, Äthylendiamin, Tris-(hydroxymethyl)-aminomethan oder Benzyl-trimethylammoniumhydroxid. Die genannten Verbindungen der Formel (I) bilden Säureadditionssalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z.B. starke Mineralsäuren, wie Halogenwasserstoffsäuren, z.B. Chlorwasserstoff- oder Bromwasserstoffsäure, Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol-, Milch-, Äpfel-, Wein-, Glucon-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl, Pamoe-, Nikotin-, Methansulfon-, Äthansulfon-, Hydroxyäthansulfon-, Benzolsulfon-, Toluolsulfon-, Naphthalinsulfon-, Sulfanil- oder Cyclohexylsulfaminsäure; oder die Ascorbinsäure.

Die Verbindungen der Erfindung zeigen wertvolle pharmakologische Eigenschaften, z.B. kardiovaskulare Effekte, durch selektive Hemmung der Thromboxan-Ausschüttung in Säugern. Diese Hemmung kommt durch selektive Verminderung der Thromboxan-Synthetase zustande. Die Verbindungen sind daher nützlich in der Behandlung von Krankheiten, welche auf Thromboxan-Synthetase-Hemmung in Säugern, einschliesslich Menschen, ansprechen.

Diese Wirkungen können durch in vitro Versuche oder in vivo Tierversuche, vorzugsweise an Säugetieren, z.B. Meerschweinchen, Mäusen, Ratten, Katzen, Hunden oder Affen nachgewiesen werden. Die genannten Verbindungen können ihnen enteral oder parenteral, vorzugsweise oral oder subkutan, intravenös oder intraperitoneal, z.B. durch Gelatinekapseln oder in Form von Stärke enthaltenden Suspensionen oder wässrigen Lösungen, verabreicht werden. Die verwendete Dosis kann in einem Bereich von ungefähr zwischen 0,01 und 100 mg/kg/Tag, vorzugsweise ungefähr 0,05 und 50 mg/kg/Tag, insbesondere ungefähr 0,1 und 25 mg/kg/Tag liegen.

Die in vitro Hemmung des Thromboxan-Synthetase-Enzyms kann analog zu der Methode von Sun, Biochem. Biophys. Res. Comm. 74, 1432 (1977) nachgewiesen werden. Das Testverfahren wird wie folgt durchgeführt: $^{14}C$-Arachidonsäure wird mit einem Enzymgemisch-Präparat, bestehend aus solubilisierter und partiell gereinigter Prostaglandin-cyclooxygenase von Schaf-Samenblasen und aus einem rohen Mikrosomen-Präparat von Thromboxan-Synthetase von lysierten menschlichen Blutplättchen, inkubiert. Die Testverbindung (gelöst in einem Puffer, oder falls nötig, in wenig Äthanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch von Prostaglandin $F_2\alpha$ und $F_2\beta$ [$PGF_2(\alpha+\beta)$] reduziert. Die radioaktiven Produkte und das überschüssige Substrat werden mit Essigsäureäthylester extrahiert und der Extrakt wird zur Trockene eingedampft. Der Rückstand wird in Aceton gelöst, auf Dünnschichtplatten tropfenweise aufgetragen und mit einem Lösungsmittelsystem von Toluol:Aceton:Eisessig [100:100:3 (Volumen)] chromatographiert. Die radioaktiven Zonen werden lokalisiert. Die Zonen von Thromboxan $B_2$ ($TxB_2$) und $PGF_2\alpha+\beta$ werden in Szintillationsröhrchen für Flüssigkeiten übertragen und gezählt. Der Quotient der Zahlenwerte von $TxB_2/PF_2\alpha+\beta$ wird für jede Konzentration der Testverbindung berechnet und die $IC_{50}$-Werte werden graphisch ermittelt. Dieser Wert ist die Konzentration der Testverbindung, bei welcher der Quotient von $TxB_2/PGF_2\alpha+\beta$ auf 50% des Kontrollwertes reduziert wird.

Die in vitro Wirkung auf Prostaglandin-cyclooxygenase wird gemäss einer Modifikation der Methode von Takeguchi et al, welche in Biochemistry 10, 2372 (1971) beschrieben ist, gemessen. Das Testverfahren ist wie folgt:

Lyophilisierte Samenblasen-Mikrosomen von Schafen werden als Enzympräparat für die Prostaglandin-Synthese verwendet. Es wird die Umwandlung von $^{14}C$-Arachidonsäure in $PGE_2$ gemessen. Die Testverbindungen (gelöst in einem Puffer, oder wenn nötig in wenig Äthanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen werden in Szintillationsröhrchen für Flüssigkeiten übertragen und ihre Radioaktivität gezählt. Die $IC_{50}$-Werte der Hemmung werden graphisch ermittelt. Dieser Wert bedeutet die Konzentration der Testverbindung, welche die Menge des synthetisierten $PGE_2$ um 50% reduziert.

Die in vitro Wirkung auf Prostacyclin-($PGI_2$)-Synthetase wird analog zu der Methode von Sun et al., Prostaglandins 14, 1055 (1977) gemessen. Das Testverfahren ist wie folgt:

$^{14}C$-Arachidonsäure wird mit einem Enzymgemisch, bestehend aus solubilisierter und partiell gereinigter Prostaglandin-cyclo-oxygenase von Schaf-Samenblasen und aus rohem $PGI_2$-Synthetase in der Form einer Mikrosomen-Fraktion von Aorten von Rindern, inkubiert.

Die Testverbindung (gelöst in einem Puffer, oder wenn nötig, in wenig Äthanol) wird in das Inkubationsmedium gegeben. Das Reaktionsgemisch wird in 100 mMolarem Tris HCl (pH 7,5) 30 Minuten bei 37° inkubiert, auf den pH-Wert 3 angesäuert und mit Essigsäureäthylester extrahiert. Der Extrakt wird zur Trockene eingedampft, der Rückstand in Aceton gelöst auf Dünnschicht-Platten aufgetragen und mit einem von Sun et al. beschriebenen Lösungsmittelsystem chromatographiert. Die radioaktiven Zonen werden mit einem Detektor lokalisiert. Die dem 6-Keto-$PGF_1\alpha$ (ein stabiles Endprodukt der Prostacyclin-Biotransformation) und $PGE_2$ entsprechenden Zonen werden in Szintillationsröhrchen für Flüssigkeiten übertragen und gezählt. Der Quotient der Zahlenwerte von 6-Keto-$PGF_1\alpha/PGF_2$ wird für jede Konzentration der verwendeten Testverbindung berechnet. Die $IC_{50}$-Werte der Hemmung werden graphisch ermittelt. Dieser Wert ist die Konzentration der Testverbindung, bei welcher der Quotient von 6-Keto-$PGF_1\alpha/PGE_2$ auf 50% des Kontrollwertes reduziert wird.

Die Abnahme des Thromboxan-Plasmaspiegels wird in vivo durch Verabreichung der Testverbindung an Meerschweinchen wie folgt bestimmt: Meerschweinchen werden mit der Testsubstanz oder Trägermaterial behandelt und 2 Stunden später wird ihnen Arachidonsäure (40 mg/kg) intraperitoneal injiziert. Eine Stunde nach der Verabreichung der Arachidonsäure wird den Tieren Blut entnommen. In einer bestimmten Einzelmenge jeder Plasmaprobe wird das Thromboxan $B_2$, und aus weiteren Einzelmengen werden das 6-Keto-$PGF_1\alpha$, die stabilen Metaboliten des Thromboxans $A_2$ bzw. das Prostacyclin ($PGI_2$) bestimmt.

Die Verbindungen der Formel (I) sind sehr wirksame Thromboxan-Synthetase-Inhibitoren. Bei wirksamen Dosis-Spiegeln und darüber, wird weder das vorteilhafte Prostacyclin-Synthetasenoch das Prostaglandin-cyclooxygenase-Enzymsystem gehemmt.

Der $IC_{50}$-Wert für eine Verbindung der Erfindung, z.B. für das 5-(5-Carboxypentyl)-imidazo[1,5-a]pyridin beträgt $3\times10^{-9}$ Mol für die Thromboxan-Synthetase-Hemmung, während für die Hemmung der Prostacyclin-Synthetase und der Prostacyclin-cyclo-oxygenase der IC-Wert jeweils grösser als $1\times10^{-4}$ Mol ist.

Eine Verbindung der Erfindung, z.B. das 5-(5-Carboxypentyl)-imidazo[1,5-a]pyridin vermindert auch den Plasmaspiegel von Thromboxan $B_2$ in Meerschweinchen, bei einer so niedrigen oralen Dosis wie 0,25 mg/kg, um mehr als 50%. Bei dieser oder höheren oralen Dosen wird keine signifikante Abnahme des Prostacyclins festgestellt.

Aufgrund der vorhergenannten vorteilhaften Eigenschaften sind die Verbindungen der Erfindung für Säuger, einschliesslich Menschen, als spezifische therapeutische Mittel sehr wertvoll. So reduziert z.B. bei der Thromboembolie diese spezifische Hemmwirkung des Thromboxan-Synthetase-Enzyms die von der Arachidonsäure hervorgerufene Aggregation von Blutplättchen, welche bei der Entstehung von Blutgerinseln eine Rolle spielt. Experimentell wird die Verlängerung der Blutungszeit an der Ratte als Zeichen für die günstige antithrombotische Wirkung angesehen. Die Imidazo[1,5-a]pyridine der vorliegenden Erfindung verlängern die Blutungszeit. So weist z.B. das 5-(5-Carboxypentyl)-imidazo[1,5-a]pyridin diese Wirkung bei intraperitonealer Verabreichung an Ratten bei einer Dosis von ungefähr 1 mg/kg oder darunter, auf.

Auf die günstigen Wirkungen bei Atmungsstörungen ist die Tatsache hinweisend, dass die Verbindungen der vorliegenden Erfindung Schutz gegen den plötzlichen Tod, der aufgrund der durch die Arachidonsäure hervorgerufenen Lungenobstruktion eintritt, gewähren. So schützt z.B. das 5-(5-Carboxypentyl)-imidazo[1,5-a]pyridin die Maus gegen den plötzlichen Tod bei einer

oralen Verabreichung einer Dosis von 100 mg/kg.

Die Verbindungen der vorliegenden Erfindung werden nach an sich bekannten Methoden, vorzugsweise dadurch hergestellt, dass man entweder

a) eine Verbindung der Formel (VI)

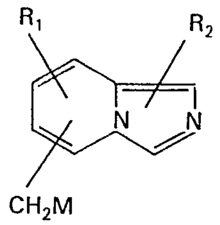

VI

worin M ein Alkalimetall bedeutet, jedes der Symbole $R_1$ und $R_2$ für Wasserstoff oder $C_1$-$C_7$-Alkyl steht, mit einem reaktionsfähigen funktionellen Derivat einer Verbindung der Formel (VII)

HO-A-B′ VII

worin A die oben angegebene Bedeutung hat, B′ Carboxy, Trialkoxymethyl, unsubstituiertes, Mono- oder Di-($C_1$-$C_7$-alkyl) substituiertes Carbamoyl, veräthertes Hydroxymethyl oder Halogenmethyl, ferner auch Cyan, bedeutet, umsetzt, und, eine erhaltene Verbindung der Formel (Ia)

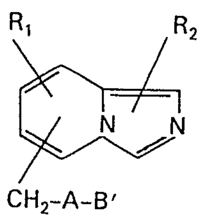

Ia

worin sich B′ von B unterscheidet, in die Verbindung der Formel (I) umwandelt, und, wenn erwünscht, eine erhaltene Verbindung der Formel (I) in eine andere Verbindung der Erfindung umwandelt.

Reaktionsfähige organometallische Verbindungen der Formel (VI), worin M ein Alkalimetallatom bedeutet, können durch Metallisierung von geeigneten Methyl-substituierten Imidazo[1,5-a]pyridinen erhalten werden. So wird, z.B. das gemäss Journal of Organic Chemistry 40, 1210 (1975) hergestellte 5-Methylimidazol[1,5-a]pyridin mit einem reaktionsfähigen Metallisierungsmittel, z.B. mit Butyllithium oder Lithium-diisopropylamid, in einem inerten Lösungsmittel, z.B. Tetrahydrofuran, bei einer Temperatur unter Zimmertemperatur, vorzugsweise bei ungefähr -50°, umgesetzt.

Die Kondensation eines Zwischenproduktes der Formel (VI) mit einem reaktionsfähigen funktionellen Derivat einer Verbindung der Formel (VII) wird vorzugsweise in einem Temperaturbereich von ungefähr -75° bis +50° vorgenommen. Wenn B′ Carboxy oder Mono-(niederalkyl)-carbamoyl bedeutet, dann wird zuerst das geeignete Metallsalz, z.B. das Lithiumsalz des reaktionsfähigen funktionellen Derivates der entsprechenden Verbindung der Formel (VII) hergestellt und dieses mit dem Zwischenprodukt (VI) umgesetzt.

Ein weiteres Verfahren zur Herstellung von Verbindungen der Formel (I) besteht darin, dass man

b) eine Verbindung der Formel (VIII)

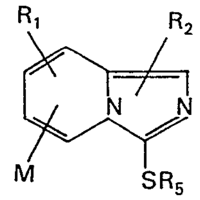

VIII

worin M ein Alkalimetall bedeutet, jedes der Symbole $R_1$ und $R_2$ für Wasserstoff oder $C_1$-$C_7$-Alkyl steht, und $R_5$ $C_1$-$C_7$-Alkyl bedeutet, mit einem reaktionsfähigen funktionellen Derivat einer Verbindung der Formel (IX)

$HOCH_2$-A-B′ IX

worin A die oben angegebene Bedeutung hat, B′ Carboxy, Trialkoxymethyl, unsubstituiertes, Mono- oder Di-($C_1$-$C_7$-alkyl) substituiertes Carbamoyl, veräthertes Hydroxymethyl oder Halogenmethyl, ferner auch Cyan, bedeutet, umsetzt, in einer erhaltenen Verbindung, worin sich B′ von B unterscheidet, die Gruppe B′ in B umwandelt, die erhaltene Verbindung desulfuriert, und, wenn erwünscht, eine erhaltene Verbindung der Formel (I) in eine andere Verbindung der Erfindung umwandelt.

Die Herstellung des organometallischen Zwischenprodukts (VIII) und die nachfolgende Kondensation werden wie oben und in Tetrahedron Letters 21, 2195-6 (1980) beschrieben, durchgeführt. Die Entschwefelung wird vorzugsweise mit einem Entschwefelungskatalysator wie Raney-Nickel in einem Lösungsmittel, z.B. Äthanol, vorzugsweise bei erhöhter Temperatur vorgenommen.

Weiter können die Verbindungen der Formel (I) dadurch hergestellt werden, dass man

c) unter basischer Katalyse eine Verbindung der allgemeinen Formel (X)

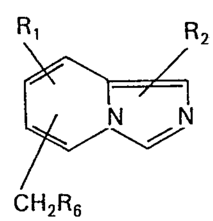

X

worin jedes der Symbole $R_1$ und $R_2$ Wasserstoff oder $C_1$-$C_7$-Alkyl bedeutet, $R_6$ für $C_1$-$C_7$-Alkoxycarbonyl oder Cyan steht, mit einem reaktionsfähigen funktionellen Derivat einer Verbindung der Formel (VII)

HO-A-B′ VII

worin A die vorher angegebene Bedeutung hat, B′ für Carboxy, Trialkoxymethyl, unsubstituiertes, Mono- oder Di-($C_1$-$C_7$-alkyl) substituiertes

Carbamoyl, veräthertes Hydroxymethyl oder Halogenmethyl, ferner auch Cyan, steht, umsetzt, die Gruppe $R_6$ abspaltet, eine erhaltene Verbindung, worin sich B′ von B unterscheidet, in eine Verbindung der Formel (I) umwandelt, und, wenn erwünscht, eine erhaltene Verbindung der Formel (I) in eine andere Verbindung der Erfindung umwandelt.

Die Zwischenprodukte der Formel (X) können durch Behandlung der oben genannten Verbindungen der Formel (VI), z.B. mit Kohlendioxid und Veresterung der erhaltenen Carbonsäure, oder durch Umsetzung mit einem Di-$C_1$-$C_7$-alkyl)-carbonat oder mit einem Cyanhalogenid, hergestellt werden.

Ein anderes Verfahren zur Herstellung von Verbindungen der Formel (I) besteht darin, dass man

d) eine Verbindung der Formel (XI)

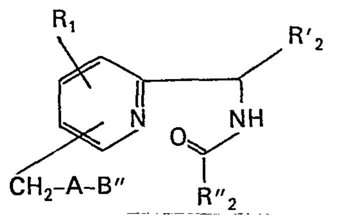

XI

worin jedes der Symbole $R_1$, $R_2'$ und $R_2''$ Wasserstoff oder $C_1$-$C_7$-Alkyl bedeutet, A die oben angegebene Bedeutung hat, und B″ für Carboxy, $C_1$-$C_7$-Alkoxycarbonyl, unsubstituiertes, Mono- oder Di-($C_1$-$C_7$-alkyl) substituiertes Carbamoyl, Cyan, Hydroxymethyl, $C_1$-$C_7$-Alkanoyloxymethyl, veräthertes Hydroxymethyl oder Halomethyl steht, zu einer Verbindung der Formel (Ib)

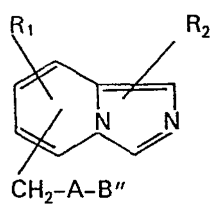

Ib

ringschliesst, eine erhaltene Verbindung, worin sich B″ von B unterscheidet, in eine Verbindung der Formel (I) umwandelt, und, wenn erwünscht, eine erhaltene Verbindung der Formel (I) in eine andere Verbindung der Erfindung umwandelt.

Der Ringschluss des Amids der Formel (XI) wird vorzugsweise mit einer Lewissäure, z.B. Polyphosphorsäure, Phosphoroxychlorid oder Polyphosphat-ester, gegebenenfalls in einem inerten Lösungsmittel, z.B. Toluol, in einem Temperaturbereich zwischen 25° bis 150°, vorzugsweise 50° bis 120°, durchgeführt.

Die Amide der Formel (XI) werden durch Acylierung einer Verbindung der Formel (XII)

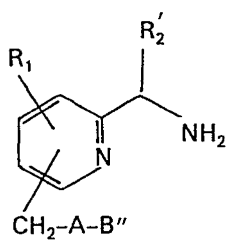

XII

worin jedes der Symbole $R_1$, $R_2'$, A und B″ die oben angegebene Bedeutung haben, mit einer Carbonsäure der Formel (XIII)

$R_2''$-COOH XIII

worin $R_2''$ die oben angegebene Bedeutung hat, oder mit einem reaktionsfähigen funktionellen Derivat davon, hergestellt.

Reaktionsfähige funktionelle Derivate von Verbindungen (XIII) sind vorzugsweise Säurehalogenide, einfache oder gemischte Anhydride, z.B. das Säurechlorid, das Säureanhydrid $(R_2''CO)_2O$, oder ein gemischtes Anhydrid. Dieses kann von einem Niederalkoxycarbonyl-halogenid, z.B. Chlorameisensäureäthylester, oder von einem gehinderten Niederalkanoylhalogenid, z.B. vom Pivaloylchlorid, nach an sich bekannter Methode, hergestellt werden.

Die Kondensation von Verbindungen (XII) und (XIII) [Acylierung von Verbindungen (XII)] verläuft entweder spontan, z.B. durch Erhitzen mit Ameisensäure, oder in Gegenwart von Kondensationsmitteln, wie disubstituierten Carbodiimiden, z.B. Dicyclohexylcarbodiimid.

Die Acylierung von Verbindungen (XII) mit einem reaktionsfähigen funktionellen Derivat von (XIII) z.B. mit Acetylchlorid oder Essigsäureanhydrid, wird vorzugsweise in Gegenwart einer organischen oder anorganischen Base, z.B. Kaliumcarbonat oder Triäthylamin durchgeführt.

Die Amine der Formel (XII) können z.B. aus entsprechend substituierten 2-(Cyan oder Hydroxyimino-niederalkyl)-pyridinen durch Reduktion, z.B. durch Hydrierung in Gegenwart eines Katalysators wie Palladium auf Kohle, oder durch Behandlung mit einem chemischen Reduktionsmittel, z.B. Boran oder Natriumcyanborhydrid erhalten werden. Das Reduktionsmittel wird je nach dem Typus der anderen, im Molekül vorhandenen funktionellen Gruppen gewählt. Die Verbindungen der Formel (XII) können auch durch Aminierung der entsprechend substituierten und reaktionsfähigen veresterten 2-(Hydroxymethyl)-pyridinen erhalten werden.

Ein weiteres Verfahren zur Herstellung von Verbindungen der Formel (I), worin A eine geradkettige oder verzweigte Alkylenkette bedeutet, besteht darin, dass man

e) eine Verbindung der Formel (XIV)

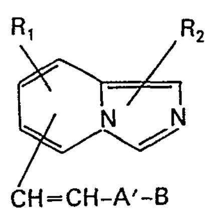

XIV

worin A′ einen Alkylen-, Alkenylen- oder Alkynylenrest mit höchstens 11 Kohlenstoffatomen bedeutet, hydriert, und, wenn erwünscht, eine erhaltene Verbindung der Formel (I) in eine andere Verbindung der Erfindung umwandelt.

Die Reaktion wird nach an sich bekannten Methoden, z.B. vorzugsweise mit Wasserstoff in Gegenwart von einem Katalysator, z.B. Palladium, durchgeführt.

Die Ausgangsstoffe der Formel (XIV) können z.B. durch Kondensation einer Verbindung der Formel (XV)

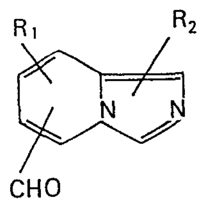

XV

[erhalten z.B. durch Umsetzung einer Verbindung der Formel (VIII) mit Dimethylformamid und nachfolgende Desulfurierung mit Raney-Nikkel] in einer Wittig-Reaktion, z.B. mit einem Triniederalkyl-4-phosphonocrotonat, in Gegenwart einer starken Base, z.B. Natriumhydrid, hergestellt werden.

Ein weiteres Verfahren für die Herstellung von Verbindungen der Formel (I), vorzugsweise von solchen, worin A einen Alkylenrest bedeutet, besteht darin, dass man

f) in einer Verbindung der Formel (XVI)

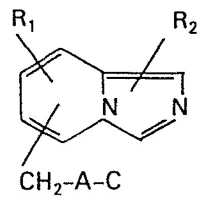

XVI

oder in ihrem 5,6,7,8-Tetrahydroderivat, worin $R_1$, $R_2$ und A die vorher angegebene Bedeutung haben und C einen in eine Carboxygruppe überführbaren Rest bedeutet, die Gruppe C, gegebenenfalls unter Verlängerung der Kette A innerhalb ihrer Definition, in die Carboxygruppe überführt, und, wenn erwünscht, eine erhaltene Verbindung der Formel I in eine andere Verbindung der Erfindung umwandelt.

In eine Carboxygruppe überführbare Reste sind z.B. veresterte Carboxygruppen, anhydrisierte Carboxygruppen, inklusive entsprechende Gruppen von asymmetrischen und inneren Anhydriden, amidierte Carboxygruppen, Cyan, Amidinogruppen, inklusive cyclische Amidinogruppen, wie 5-Tetrazolyl, Iminoäthergruppen, inklusive cyclische Iminoäthergruppen, wie z.B. durch Niederalkyl substituierte 2-Oxazolinyl- oder Dihydro-2-oxazolinylgruppen, ferner Hydroxymethyl, veräthertes Hydroxymethyl, Niederalkanoyloxymethyl, Trialkoxymethyl, Acetyl, Trihaloacetyl, Halomethyl, Carboxycarbonyl (COCOOH), Methyl, Formyl (CHO), Di-niederalkoxymethyl, Alkylendioxymethyl, Vinyl oder Diazoacetyl. Bei der Umwandlung in die Carboxygruppe kann gleichzeitig die Kette A innerhalb ihrer Definition verlängert werden.

Die Überführung in die Carboxygruppe wird nach an sich bekannten Methoden, wie hier oder in den Beispielen beschrieben, z.B. solvolytisch, wie hydrolytisch oder acidolytisch, oder reduktiv (veresterte Carboxygruppen) vorgenommen. So werden z.B. Trichloräthyl oder 2-Jodäthylester durch Reduktion, z.B. mit Zink und einer Carbonsäure in Gegenwart von Wasser in die Carbonsäure überführt. Benzylester oder Nitro-benzylester können durch katalytische Hydrierung, letztere auch mit chemischen Reduktionsmitteln, z.B. Natriumdithionit oder mit Zink und einer Carbonsäure, in die Carboxygruppe umgewandelt werden. Ferner können z.B. tert.-Butylester auch z.B. mit Trifluoressigsäure gespalten werden.

Bei der Reduktion der Gruppe C kann eine Alkenylen- oder Alkinylenkette A in die entsprechende Alkylenkette umgewandelt werden.

Weiter können Verbindungen der Formel (XVI) worin C Acetyl bedeutet, oxidativ zu den entsprechenden Verbindungen der Formel (I), worin B Carboxy bedeutet, gespalten werden. Zuerst wird der Ausgangsstoff in eine Verbindung (XVI), worin C Trihaloacetyl, z.B. Tribrom- oder Trijodacetyl bedeutet, z.B. durch Behandlung mit Natrium-hypobromit umgewandelt und nachfolgend z.B. mit einer wässerigen Base, z.B. Natriumhydroxid, gespalten.

Ausgangsstoffe der Formel (XVI), in welchen C Acetyl bedeutet, können ausgehend von Verbindungen Ib, worin B′ Halomethyl bedeutet, durch Behandlung mit einem Acetessigsäure-niederalkylester, z.B. Acetessigsäure-äthylester, in Gegenwart einer Base, z.B. Natriumhydrid und nachfolgende Hydrolyse mit einer starken Base, z.B. mit wässerigem Natriumhydroxid, hergestellt werden.

Ausgangsstoffe der Formel (XVI), worin C Carboxycarbonyl (COCOOH) bedeutet, werden durch thermische Behandlung oder durch Oxidation in die Verbindungen der Formel (I), worin B Carboxy bedeutet, überführt. Der Ausgangsstoff wird dabei auf eine erhöhte Temperatur, z.B. ungefähr 200°, in Gegenwart von Glaspulver erhitzt, oder z.B. mit Wasserstoffsuperoxid in Gegenwart eines basischen Mittels, z.B. Natriumhydroxid, behandelt.

Die Ausgangsstoffe der Formel (XVI), worin C COCOOH bedeutet, können z.B. durch Kondensation einer Verbindung der Formel Ia, worin B′ Halomethyl bedeutet, mit z.B. 2-Äthoxycarbonyl-1,3-dithian, und nachfolgende oxidative Hydrolyse, z.B. mit Bromsuccinimid in wässerigem Aceton und dann durch Behandlung mit verdünntem wässerigem Natriumhydroxid, erhalten werden.

Verbindungen der Formel (XVI), worin C Formyl, Di-niederalkoxy-methyl oder Alkylendioxymethyl (Formyl geschützt in der Form eines Acetals), z.B. Dimethylacetal bedeutet, werden z.B. mit Silbernitrat oder Ozon zu den Verbindungen der Formel I, worin B Carboxy bedeutet, oxidiert.

Die als Ausgangsstoffe verwendeten Carboxaldehyde, d.h. Verbindungen der Formel (XVI), worin C Formyl bedeutet, werden durch Oxidation von Verbindungen der Formel (I) oder (Ia), worin B bzw. B′ Hydroxymethyl bzw. Halomethyl bedeutet, mit z.B. Dimethylsulfoxid und einem Katalysator, z.B. einem Gemisch von Triäthylamin und Silbertetrafluoroborat, hergestellt. Die

erhaltenen Carboxaldehyde können in die entsprechenden Acetale, d.h. zu Verbindungen der Formel (XVI), worin C Di-niederalkoxymethyl oder Alkylendioxymethyl, z.B. ein Diomethylacetal ist, durch Säure katalysierte Kondensation mit einem Alkohol, z.B. Methanol, übergeführt werden.

Verbindungen der Formel (I), worin B Carboxy bedeutet, können durch die wohlbekannte Arndt-Eistert-Synthese zu Verbindungen der Formel (I), worin B Carboxy ist und die Kette ein Kohlenstoffatom mehr enthält, umgewandelt werden. Es wird insbesondere ein reaktionsfähiges funktionelles Derivat der als Ausgangsstoff verwendeten Carbonsäure, z.B. das Säurechlorid mit Diazomethan, z.B. in Diäthyläther behandelt, wobei man eine Verbindung der Formel XVI, worin C Diazoacetyl bedeutet, erhält. Umwandlung mit z.B. Silberoxid ergibt die genannte Carbonsäure der Formel I, worin die Kette A ein Kohlenstoffatom mehr enthält.

Verbindungen der Formel (XVI), worin C Vinyl bedeutet, werden in die Verbindungen der Formel (I), worin B Carboxy ist, zuerst durch Ozonolyse in die Verbindungen der Formel XVI, worin C Formyl bedeutet, umgewandelt. Diese werden dann zu den Verbindungen der Formel (I), worin B Carboxy bedeutet, oxidiert.

Ausgangsstoffe der Formel (XVI), worin C Vinyl bedeutet, können auch mit Nickelcarbonyl und Kohlenmonoxid unter Hochdruck behandelt werden, wobei man Verbindungen der Formel (I), worin B Carboxy bedeutet und die Kette A um ein Kohlenstoffatom verlängert wird, erhält.

Die einzelnen Definitionen in den vorher beschriebenen Verfahren haben die folgenden Bedeutungen:

In einem reaktionsfähigen funktionellen Derivat eines Alkohols der Formel (VII) und (IX) ist die Hydroxygruppe z.B. mit einer starken anorganischen Säure oder organischen Sulfonsäure, vor allem mit einer Halogenwasserstoffsäure, z.B. Chlorwasserstoff-, Bromwasserstoff- oder Jodwasserstoffsäure, einer aliphatischen oder aromatischen Sulfonsäure, z.B. Methansulfon- oder p-Toluolsulfonsäure, verestert. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Veräthertes Hydroxymethyl bedeutet vorzugsweise tertiäres Niederalkoxymethyl, niederes Alkoxy-alkoxymethyl, z.B. Methoxymethoxymethyl, 2-Oxa- oder 2-Thiacycloalkoxymethyl, insbesondere 2-Tetrahydropyranyloxymethyl.

Halogenmethyl bedeutet insbesondere Chlormethyl, aber auch Brommethyl oder Jodmethyl.

Niederalkanoyloxymethyl bedeutet vorzugsweise Acetoxymethyl.

Ein Alkalimetall ist vorzugsweise Lithium, es kann aber auch Kalium oder Natrium sein.

Die unerlässlichen Schritte für die Umwandlung einer erhaltenen Verbindung, worin sich B′ oder B″ von B unterscheidet, in eine Verbindung der Formel (I), bzw. die fakultativen Umwandlungen eines erhaltenen Produkts der Formel (I) in eine andere Verbindung der Erfindung werden nach an sich bekannten chemischen Methoden durchgeführt.

Die Hydrolyse von Zwischenprodukten, in welchen B′ Trialkoxymethyl bedeutet, zu Verbindungen der Formel (I), in welchen B Carboxy ist, wird vorzugsweise mit anorganischen Säuren, wie Halogenwasserstoff- oder Schwefelsäure vorgenommen. Die Hydrolyse von Zwischenprodukten, in welchen B′ veräthertes Hydroxymethyl bedeutet, zu Verbindungen der Formel I, in welchen B Hydroxymethyl ist, wird vorzugsweise mit wässerigen Lösungen von anorganischen Säuren, z.B. einer Halogenwasserstoffsäure, durchgeführt.

Zwischenprodukte der Formel (Ia) oder (Ib), in welchen B′ oder B″ Halogenmethyl bedeutet, können vorzugsweise mit einem Metallcyanid, z.B. Kaliumcyanid, in an sich bekannter Weise umgesetzt werden. Man erhält dabei Verbindungen der Formel (I), in welchen die Kette um ein Kohlenstoffatom verlängert ist und B für Cyan steht. Diese können ihrerseits nach an sich bekannten Methoden in Verbindungen der Formel (I), worin B Carboxy, Alkoxycarbonyl oder Carbamoyl bedeutet, übergeführt werden. So können Verbindungen der Formel (I), worin B Cyan bedeutet (Nitrile) in die Verbindungen der Formel (I), in welchen B für Carboxy steht, durch Hydrolyse mit anorganischen Säuren, z.B. einer Halogenwasserstoffsäure, wie Chlorwasserstoffsäure oder Schwefelsäure, in wässerigen Lösungen oder vorzugsweise durch Hydrolyse mit wässerigen Alkalimetallhydroxiden, z.B. Kaliumhydroxiden, z.B. Kaliumhydroxid, vorzugsweise bei Rückflusstemperatur, umgewandelt werden.

Die Umwandlung der genannten Nitrile in die Verbindungen der Formel (I), worin B $C_1$-$C_7$-Alkoxycarbonyl bedeutet, wird vorzugsweise durch Behandlung mit einem $C_1$-$C_7$-Alkanol, z.B. wasserfreiem Äthanol, in Gegenwart einer starken Säure, z.B. Chlorwasserstoffsäure, vorzugsweise bei Rückflusstemperatur, und nachfolgende vorsichtige Hydrolyse mit Wasser, durchgeführt.

Weiter wird die Umwandlung der genannten Nitrile in die Verbindungen der Formel (I), worin B Carbamoyl bedeutet, vorzugsweise durch Behandlung mit einem Alkalimetallhydroxid, z.B. verdünntem Natriumhydroxid und Wasserstoffsuperoxid, vorzugsweise bei Raumtemperatur durchgeführt.

Ferner können die Zwischenprodukte der Formel (Ia), (Ib) oder (XVI), in welchen B′, B″ oder C Halomethyl, z.B. Chlormethyl ist, in die Verbindungen der Formel I, worin B Carboxy ist und die Kettenlänge um zwei Kohlenstoffatome verlängert wird, wie folgt umgewandelt werden: Zuerst wird durch Behandlung mit z.B. einem Malonsäure-di-niederalkylester, wie mit Malonsäure-diäthylester, in Gegenwart einer Base, z.B. Kaliumcarbonat oder Natriumäthoxid, in einem Lösungsmittel, z.B. Dimethylformamid, vorzugsweise in einem Temperaturbereich zwischen 50

und 100°, ein substituierter Malonsäure-diniederalkylester hergestellt. Dieser wird vorzugsweise mit einer wässerigen Base, z.B. verdünntem Natriumhydroxid, zu der entsprechenden Malonsäure hydrolysiert und nach Standardbedingungen, z.B. durch Erhitzen in Chloroform, decarboxyliert. Ersetzt man den Malonsäure-diniederalkylester durch einen Cyanessigsäureniederalkylester, so erhält man die entsprechende Verbindung, worin B Cyan bedeutet.

Verbindungen der Formel (Ia) und (Ib), worin B′ und B″ Halomethyl bedeutet können in die entsprechenden metallorganischen Zwischenprodukte, z.B. Kupfer- oder Magnesiumderivate nach an sich bekannten Methoden umgewandelt werden.

Kondensation z.B. eines erhaltenen organischen Magnesium-(Grignard)-Reagenses, z.B. mit einer Verbindung der Formel (Ia), worin B′ Halomagnesium bedeutet, ergibt z.B. $CH_2MgCl$, und mit Kohlendioxid eine Verbindung der Formel (I) entsteht, worin B Carboxy bedeutet und die Kette um ein Kohlenstoffatom verlängert wird.

Kondensation des genannten Grignard-Reagenses mit z.B. einem Halogenessigsäureniederalkylester, z.B. Bromessigsäure-äthylester ergibt eine Verbindung der Formel (I), worin B Niederalkoxycarbonyl bedeutet und die Kette um 2 Kohlenstoffatome verlängert wird.

Das genannte Grignard-Reagens kann in Gegenwart eines Kupfer-I-halogenids, z.B. Kupfer-I-chlorids, mit einer α,β-ungesättigten Säure oder einem Ester, z.B. mit Propiolsäure oder Acrylsäure, kondensiert werden, wobei man eine Verbindung der Formel (I) erhält, worin B Carboxy oder Niederalkoxycarbonyl bedeutet und die Kette durch 3 Kohlenstoffatome verlängert wird.

Ferner können Verbindungen der Formel (Ia) und (Ib), worin B′ und B″ Halomethyl bedeutet mit z.B. dem 3-Lithio-Derivat der Propiolsäure (hergestellt in situ aus Propiolsäure und z.B. Lithium-diisopropylamid) kondensiert werden, wobei man eine Verbindung der Formel (I) erhält, in welcher A ein terminales Alkynylen bedeutet, B für Carboxy steht und die Kettenlänge um 3 Kohlenstoffatome verlängert wird.

Verbindungen der Erfindung, worin A einen geraden und verzweigten Alkenylenrest bedeutet und eine terminale Doppelbindung aufweist, können auch aus den Zwischenprodukten der Formel (Ia) oder (Ib), worin B′ oder B″ Halomethyl bedeutet, hergestellt werden. So werden z.B. diese Zwischenprodukte zuerst mit z.B. einem α-(Aryl- oder Alkyl)-thioessigsäure-niederalkylester, wie α-(Phenylthio)-essigsäure-äthylester behandelt. Die Reaktion wird in Gegenwart einer starken Base, z.B. Natriumhydroxid durchgeführt. Die nachfolgende Oxidation des erhaltenen α-Arylthio- oder α-Alkylthio substituierten Esters zu dem α-Arylsulfinyl- oder α-Alkylsulfinylester, mit z.B. Natriumperjodat, und nachfolgende durch Hitze ausgelöste Eliminierung, z.B. durch Kochen in Xylol unter Rückfluss, ergibt eine Verbindung der allgemeinen Formel (I) (einen α,β-ungesättigten Ester), worin A Alkenylen bedeutet und B z.B. für $C_1$-$C_7$-Alkoxycarbonyl steht, und die Länge der Kette um 2 Kohlenstoffatome verlängert wird. Ähnlich können Verbindungen der Formel Ia, worin B′ Halomethyl bedeutet, zuerst zu den entsprechenden Carboxaldehyden, z.B. mit Dimethylsulfoxid in Gegenwart von Triäthylamin und Silber-tetrafluoroborat umgewandelt werden. Die nachfolgende Wittig-Kondensation, z.B. mit (Triphenylphosphoranyliden)-essigsäureäthylester, ergibt auch die vorher genannten α,β-ungesättigten Ester.

Verbindungen der Formel (I), worin B $C_1$-$C_7$-Alkoxycarbonyl bedeutet, können mit Ammoniak, Mono- oder Di-niederalkylaminen, z.B. Methylamin, oder Dimethylamin, in einem inerten Lösungsmittel, z.B. Niederalkanol, wie Butanol, gegebenenfalls bei erhöhten Temperaturen, zu Verbindungen der Formel (I), in welchen B unsubstituiertes Mono- oder Di-(niederalkyl) substituiertes Carbamoyl bedeutet, amidiert werden.

Verbindungen der Formel (I), in welchen B für unsubstituiertes Carbamoyl steht, können zu den entsprechenden Nitrilen in an sich bekannter Weise, z.B. durch Behandlung mit Triphenylphosphin oder Thionylchlorid, in einem inerten Lösungsmittel, z.B. Toluol, dehydratisiert werden.

Die Umwandlung von Verbindungen der Formel (I), in welchen B $C_1$-$C_7$-Alkoxycarbonyl, Cyan, unsubstituiertes, Mono- oder Di-($C_1$-$C_7$-alkyl) substituiertes Carbamoyl bedeutet, zu Verbindungen der Formel I, in welchen B für Carboxy steht, wird vorzugsweise durch Hydrolyse mit anorganischen Säuren, z.B. mit Halogenwasserstoffsäuren oder Schwefelsäure, oder mit wässerigen Alkalien, vorzugsweise mit Alkalimetallhydroxiden, z.B. Lithium- oder Natriumhydroxid, durchgeführt.

Verbindungen der Formel (I), in welchen B Carboxy oder Niederalkoxycarbonyl bedeutet, können mit einfachen oder komplexen Leichtmetallhydriden, z.B. mit Lithiumaluminiumhydrid, Alan oder Diboran, zu Verbindungen der Formel (I), in welchen B für Hydroxymethyl steht, reduziert werden. Die Alkohole können auch durch geeignete Solvolyse von Zwischenprodukten der Formel Ia oder Ib, worin B′ und B″ Halogenmethyl bedeutet, durch Behandlung z.B. mit einem Alkalimetallhydroxid, z.B. Lithium oder Natriumhydroxid, erhalten werden.

Die vorher genannten Alkohole können ihrerseits in Verbindungen der Formel (I), worin B Carboxy bedeutet, mit konventionellen Oxidationsmitteln, vorzugsweise mit Pyridin-dichromat in Dimethylformamid bei Raumtemperatur, umgewandelt werden. Die genannten Alkohole können auch in die Verbindungen der Formel (I), worin B Carboxy bedeutet, und die Kette durch 1 Kohlenstoffatom verlängert wird, durch Behandlung mit Nickelcarbonyl und Kohlenmonoxid unter hohem Druck, umgewandelt werden.

Freie Carbonsäuren können mit $C_1$-$C_7$-Alkanolen, z.B. Äthanol in Gegenwart einer starken Säure, z.B. Schwefelsäure, vorzugsweise bei erhöhten Temperaturen, oder mit Diazo-niederalkanen,

z.B. Diazomethan in einem Lösungsmittel, z.B. Äthyläther, vorzugsweise bei Raumtemperatur, zu den entsprechenden Estern, nämlich zu solchen Verbindungen der Formel (I), worin B $C_1$-$C_7$-Alkoxycarbonyl bedeutet, verestert werden.

Weiter können die freien Carbonsäuren durch Behandlung ihrer reaktionsfähigen Zwischenprodukte, z.B. eines Acylhalogenids, wie eines Säurechlorids, oder gemischten Anhydrids, z.B. eines das von einem Halogenkohlensäureniederalkylester, z.B. Chlorameisensäure-äthylester abgeleitet ist, mit Ammoniak, Mono- oder di-niederalkylaminen, in einem inerten Lösungsmittel, z.B. Methylenchlorid, vorzugsweise in Gegenwart eines basischen Katalysators, z.B. Pyridin, in die Verbindungen der Formel I, in welchen B für unsubstituiertes, Mono- oder Di-(niederalkyl) substituiertes Carbamoyl steht, umgewandelt werden.

Verbindungen der Formel (I), worin B Mono-$C_1$-$C_7$-alkyl-carbamoyl bedeutet, können in die Verbindungen der Formel (I), in welchen B für Di-$C_1$-$C_7$-alkyl-carbamoyl steht, durch Behandlung mit einer starken Base, z.B. Natriumhydrid und dann mit einem Alkylierungsmittel, z.B. einem Niederalkylhalogenid, in einem inerten Lösungsmittel, z.B. Dimethylformamid, übergeführt werden.

Verbindungen der Formel (I) können in die entsprechenden 5,6,7,8-Tetrahydro-imidazo[1,5-a]pyridin-Verbindungen durch Reduktion mit Wasserstoff in Gegenwart von Hydrierungskatalysatoren, z.B. Palladium und einer Säure, z.B. einer Mineralsäure, wie Chlorwasserstoffsäure, in einem inerten Lösungsmittel, z.B. Äthanol, umgewandelt werden.

Weiter können Verbindungen der Formel (I), in welchen A einen geradkettigen oder verzweigten Alkynylen- oder Alkenylenrest bedeutet, durch katalytische Hydrierung, vorzugsweise unter neutralen Bedingungen, z.B. mit einem Palladium-Katalysator bei atmosphärischem Druck in einem inerten Lösungsmittel, z.B. Äthanol, in die Verbindungen der Formel I, worin A für geradkettiges oder verzweigtes Alkylen steht, umgewandelt werden.

Überdies können Verbindungen der Formel (I), worin $R_2$ Wasserstoff bedeutet, in die entsprechenden Halogenderivate durch direkte Halogenierung mit Chlor, Brom oder Jod, übergeführt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen führen.

Die Erfindung betrifft auch die neuen Ausgangsstoffe und Verfahren zu ihrer Herstellung.

Je nach der Wahl von Ausgangsstoffen und Verfahren, können die neuen Verbindungen in Form eines der möglichen Isomeren oder Gemischen von solchen, vorliegen. So können sie z.B. je nach der Anwesenheit einer Doppelbindung und nach der Anzahl der asymmetrischen Kohlenstoffatomen, als reine optische Isomeren, z.B. als Antipoden, oder als Gemische von Isomeren, z.B. als Racemate, Gemische von Diastereomeren, Gemische von Racematen oder Gemische von geometrischen Isomeren sein.

Erhaltene Gemische von Diastereomeren, Gemische von Racematen oder geometrischen Isomere können auf der Basis der physikochemischen Unterschiede der Komponenten, in an sich bekannter Weise, in die reinen Isomeren, Diastereomeren, Racematen oder geometrischen Isomeren, z.B. durch Chromatographie und/oder fraktionierte Kristallisation, getrennt werden.

Erhaltene Racemate können weiter in die optischen Antipoden in an sich bekannter Weise, z.B. durch Umkristallisation aus optisch aktiven Lösungsmitteln, durch Mikroorganismen, oder durch Umsetzung des saueren Endprodukts mit einer optisch aktiven Base, welche mit der racemischen Säure Salze bildet, aufgetrennt werden. Diese Salze können in dieser Weise, z.B. aufgrund ihrer verschiedenen Löslichkeiten, in die Diastereomeren getrennt werden. Aus den letzteren können die Antipoden durch Einwirkung von geeigneten Mitteln freigesetzt werden. Basische racemische Produkte können in analoger Weise, wie durch Trennung ihrer diastereomeren Salze, z.B. durch fraktionierte Kristallisation der d- oder l-Tartrate, in die Antipoden aufgetrennt werden.

Vorzugsweise wird von den zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxyd oder Ammoniumhydroxyd, basischen Salz, z.B. einem Alkalimetallhydroxid oder -carbonat, oder einem Kationenaustauscher, umgewandelt werden. Eine

Verbindung der Formel I, in welcher B Carboxy bedeutet, kann auch in die entsprechenden Metall- oder Ammoniumsalze übergeführt werden. Diese oder andere Salze, z.B. die Pikrate, können auch in der Reinigung von freien Basen verwendet werden. Die Basen werden in ihre Salze übergeführt, die Salze abgetrennt und die Basen aus den Salzen freigesetzt.

Infolge der engen Beziehung zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere, für die Kristallisation verwendeten Lösungsmittel einschliessen.

Die erfindungsgemässen pharmazeutischen Präparate sind solche, die sich für enterale, z.B. orale oder rektale, und parenterale Verabreichung an Säugern, inklusive Menschen, eignen. Die Präparate werden für die Behandlung oder Vorbeugung von Krankheiten, welche auf die Hemmung der Thromboxan-Synthetase, reagieren, z.B. periphäre vaskulare Krankheiten verwendet. Diese Präparate enthalten eine wirksame Dosis einer pharmakologisch aktiven Verbindung der Formel (I), oder eines pharmazeutisch verwendbaren Salzes davon, allein oder in Kombination mit einem oder mehreren pharmazeutisch annehmbaren Trägermaterialien.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, des Aktivstoffes. Einzeldosen für Säuger mit einem Gewicht von ungefähr 50–70 kg können zwischen ungefähr 10–200 mg des aktiven Bestandteils enthalten.

Die folgenden Beispiele dienen zur Illustration der Erfindung und sie sind nicht als Einschränkung ihres Umfangs aufzufassen. Temperaturen werden in Celsiusgraden angegeben, und die Angaben über Teile betreffen Gewichtsteile. Wenn nicht anders definiert, wird das Eindampfen von Lösungsmitteln unter verminderten Druck, z.B. zwischen ungefähr 15 und 100 mg/Hg, durchgeführt.

Beispiel 1:

Eine Lösung von 50 g 5-Methylimidazo[1,5-a]pyridin [J. Org. Chem. 40, 1210 (1975)] in 625 ml Tetrahydrofuran wird auf –75° vorgekühlt und unter Stickstoff mit 175 ml 2,4-normalem Butyllithium in Hexan versetzt, wobei man die Temperatur unter –53° hält. Die Lösung von 5-(Lithiomethyl)-imidazol[1,5-a]pyridin wird wieder auf –75° gekühlt und mit einer Lösung von 121,8 g 5-Brom-1,1,1-triäthoxypentan in 125 ml Tetrahydrofuran rasch versetzt, wobei die Temperatur auf –60° steigt. Man lässt das Reaktionsgemisch innerhalb 45 Minuten sich auf –4° erwärmen und dampft es praktisch zur Trockene ein. Der Rückstand wird zwischen 500 ml Äthyläther und 240 ml 3-normaler Chlorwasserstoffsäure verteilt. Die Ätherlösung wird weiter zweimal mit 60 ml 3-normaler Chlorwasserstoffsäure extrahiert. Die vereinigten wässerigen Extrakte werden mit 100 ml konz. Ammoniumhydroxid basisch gemacht und zweimal mit 200 ml Äthyläther extrahiert. Der Ätherextrakt wird über Magnesiumsulfat getrocknet und zur Trockene eingedampft. Das erhaltene Öl wird im Hochvakuum destilliert. Man erhält das 5-(5-Äthoxycarbonylpentyl)-imidazol[1,5-a]pyridin, welches bei 180–186°/0,12 mmHg siedet.

Beispiel 2:

Eine Suspension von 26 g 5-(5-Äthoxycarbonylpentyl)-imidazo[1,5-a]pyridin in 100 ml 1-normaler wässeriger Natriumhydroxidlösung wird zwei Stunden auf dem Dampfbad erhitzt, mit 10 ml Äthanol versetzt und 45 Minuten weiter erhitzt. Das Reaktionsgemisch wird abgekühlt, mit 300 ml Äther gewaschen und die Lösung mit konz. Chlorwasserstoffsäure auf den pH-Wert 5,5 eingestellt. Das kristallisierte Produkt wird abfiltriert und mit 50 ml Wasser gewaschen. Man erhält das 5-(5-Carboxypentyl)-imidazol[1,5-a]pyridin, welches bei 144–147° schmilzt.

Beispiel 3:

a) Eine Lösung von 39,6 g 5-Bromvaleriansäure in 400 ml Tetrahydrofuran wird auf –78° gekühlt und langsam mit 93 ml 2,3-normaler Butyllithium-Lösung in Hexan versetzt, wobei man die Temperatur unter -65° hält. Die Suspension wird 20 Minuten gerührt und dann mit einer Lösung von 5-(Lithiomethyl)-imidazol[1,5-a]pyridin (hergestellt aus 26.9 g 5-Methylimidazol[1,5-a]pyridin und 93 ml 2,3-normaler n-Butyllithium-Lösung gemäss Beispiel 1) auf einmal bei -75° versetzt. Das Reaktionsgemisch wird bei -75° zwei Stunden gerührt, sich auf Zimmertemperatur erwärmen gelassen, mit 15 ml 12-normaler Chlorwasserstoffsäure behandelt und im Vakuum eingedampft.

Der Rückstand wird zwischen Wasser und Methylenchlorid verteilt, nachdem man den pH-Wert mit Natriumcarbonat auf 10 eingestellt hat. Die wässrige Lösung wird mit Chloroform weiter gewaschen, mit 12-normaler Chlorwasserstoffsäure auf den pH-Wert 1 eingestellt und wieder mit Äther und Toluol gewaschen. Der pH-Wert wird mit Natriumhydrogencarbonat auf 5,5 eingestellt, und die Extraktion mit Chloroform ergibt das rohe 5-(5-Carboxypentyl)-imidazol[1,5-a]pyridin. Eine Lösung der Säure in 30 ml Acetonitril wird mit 20 ml 5-normaler Chlorwasserstoffsäure behandelt. Nach Zugabe von 25 ml Äthyläther erhält man das kristalline 5-(5-Carboxypentyl)-imidazol[1,5-a]pyridin-hydrochlorid, welches bei 201–204° schmilzt. Das 5-(5-Carboxypentyl)-imidazol[1,5-a]pyridin (Beispiel 2) wird nach dem Neutralisieren der methanolischen Lösung mit verdünnter Natriumhydroxidlösung auf den pH-Wert 5 erhalten.

b) Ausgehend von 6-Bromhexansäure wird in analoger Weise das 5-(6-Carboxyhexyl)-imidazol[1,5-a]pyridin, welches bei 137–139° schmilzt, hergestellt.

c) In analoger Weise wird auch das 5-(7-Carboxyheptyl)-imidazol[1,5-a]pyridin ausgehend von 7-Bromheptansäure erhalten. F. 97–101°.

Beispiel 4:

Eine Lösung von 37 g 5-(5-Chlorpentyl)-imidazo[1,5-a]pyridin, 21,7 g Kaliumcyanid und 3 g Dibenzo-18-kronenäther-6 in 500 ml Acetonitril wird 20 Stunden unter Rückfluss gekocht. Das Acetonitril wird unter vermindertem Druck abgedampft, der Rückstand zwischen Wasser und Methylenchlorid verteilt und der Methylenchlorid-Extrakt zur Trockene eingedampft. Durch Behandlung einer Lösung des Rückstands in Äther mit äthanolischer Chlorwasserstoffsäure erhält man das 5-(5-Cyanpentyl)-imidazo[1,5-a]pyridin-hydrochlorid, welches bei 178–180° schmilzt.

Der Ausgangsstoff wird wie folgt hergestellt:

Eine Lösung von 30 g 1-Brom-4-chlorbutan in 20 ml trockenem Tetrahydrofuran wird zu einer Lösung von 5-(Lithiomethyl)-imidazo[1,5-a]pyridin (hergestellt gemäss Beispiel 1 aus 22 g 5--Methyl-imidazo[1,5-a]pyridin und 80 ml einer 2,3-normalen Lösung von n-Butyllithium in Hexan) gegeben, wobei man die Temperatur unter -50° hält. Das Reaktionsgemisch wird 2–3 Stunden bei -50° gerührt, auf Zimmertemperatur erwärmt, über Nacht gerührt und zur Trockene eingedampft. Die Lösung des Rückstandes in 200 ml Methylenchlorid wird mit Wasser gewaschen, über Magnesiumsulfat getrocknet und zur Trockene eingedampft. Man erhält das 5-(5-Chlorpentyl)-imidazo[1,5-a]pyridin, welches ohne weitere Reinigung verwendet wird.

Beispiel 5:

Gemäss dem im Beispiel 4 beschriebenen Verfahren wird das 5-(4-Chlorbutyl)-imidazo[1,5-a]pyridin in das 5-(4-Cyanbutyl)-imidazo[1,5-a]pyridin umgewandelt. F. 72–77°.

Beispiel 6:

In einem dem im Beispiel 4 beschriebenen analogen Verfahren wird das 3,5-Dimethylimidazo[1,5-a]pyridin (J. Het. Chem. *3*, 33 (1966)] in das 5-(5-Chlorpentyl)-3-methyl-imidazo[1,5-a]pyridin umgewandelt. F. 98–104°. Die gemäss den Bedingungen des Beispiels 4 durchgeführte Umsetzung mit Kaliumcyanid ergibt das 5-(5-Cyanpentyl)-3-methyl-imidazo[1,5-a]pyridin, welches in sein Hydrobromid durch Auflösen der freien Base in Acetonitril und Ansäuern der Lösung mit äthanolischem Bromwasserstoff übergeführt wird. Das erhaltene 5-(5-Cyanpentyl)-3-methyl-imidazo[1,5-a]pyridin-hydrobromid schmilzt bei 215–220°.

Beispiel 7:

Eine Lösung von 36 g 5-(5-Cyanpentyl)-imidazo[1,5-a]pyridin in 100 ml Methanol und 50 ml einer 45%igen wässerigen Kaliumhydroxidlösung wird unter Rückfluss 48 Stunden gekocht. Das Methanol wird unter vermindertem Druck abgedampft und der Rückstand mit Wasser versetzt. Die basische Lösung wird mit Essigsäureäthylester gewaschen und mit konz. Chlorwasserstoffsäure auf einen pH-Wert von 5,5–6 angesäuert.

Die kristallisierte Säure wird abgetrennt und aus Äthanol umkristallisiert. Man erhält das Produkt des Beispiels 2, nämlich das 5-(5-Carboxypentyl)-imidazo[1,5-a]pyridin, welches bei 142–145° schmilzt. Eine weitere Umkristallisation erhöht den Schmelzpunkt auf 144–147°.

Beispiel 8:

Die Hydrolyse des 5-(4-Cyanbutyl)-imidazo[1,5-a]pyridins gemäss Beispiel 7 ergibt das 5-(4-Carboxybutyl)-imidazo[1,5-a]pyridin, welches bei 161–163° schmilzt.

Beispiel 9:

Die Hydrolyse des 5-(5-Cyanpentyl)-3-methyl-imidazo[1,5-a]pyridins gemäss Beispiel 7 ergibt das 5-(5-Carboxypentyl)-3-methyl-imidazo[1,5-a]pyridin, welches bei 170–173° schmilzt.

Beispiel 10:

Eine Lösung von 3 g 5-(5-Cyanpentyl)-3-methyl-imidazo[1,5-a]pyridin-hydrochlorid in einem Gemisch von 20 ml Äthanol und 5 ml 1-normaler wässeriger Natriumhydroxidlösung wird mit 10 ml einer 30%igen Wasserstoffsuperoxidlösung versetzt. Das Reaktionsgemisch wird dann mit 5 ml Äthanol versetzt und mit 1-normaler Natrium-hydroxidlösung auf den pH-Wert 10 eingestellt.

Das Gemisch wird über Nacht bei Zimmertemperatur gerührt, das Äthanol unter vermindertem Druck abgedampft, der Rückstand mit Wasser versetzt und mit Methylenchlorid extrahiert. Das erhaltene Produkt wird aus Äther kristallisiert und aus Acetonitril umkristallisiert. Man erhält das 5-(5-Carbamoylpentyl)-imidazo[1,5-a]pyridin, welches bei 131–132° schmilzt.

Beispiel 11:

Eine Lösung von 3,9 g 5-(5-Äthoxycarbonylpentyl)-imidazo[1,5-a]pyridin in 40 ml n-Butanol wird mit Methylamin gesättigt und in einem Druckgefäss 56 Stunden auf dem Dampfbad erhitzt. Das Reaktionsgemisch wird zur Trockene eingedampft. Das erhaltene Produkt wird zuerst aus Äther und dann aus einem 1:1-Gemisch von Essigsäureäthylester-Äther umkristallisiert. Man erhält das 5-[5-(N-Methylcarbamoyl)-pentyl]-imidazo[1,5-a]pyridin, welches bei 118–122° schmilzt.

Beispiel 12:

Eine Lösung von 2,45 g 5-[5-(N-Methylcarbamoyl)-pentyl]-imidazo[1,5-a]pyridin in 25 ml Dimethylformamid wird mit 0,011 Mol Natriumhydrid (erhalten durch Waschen von 0,53 g einer 50%igen Natriumhydrid-Dispersion in Mineralöl mit Hexan) versetzt und auf dem Dampfbad kurz erwärmt. Die abgekühlte gelbe Lösung wird mit 1,56 g Methyljodid versetzt. Das Gemisch wird 2 Stunden bei Zimmertemperatur gerührt, mit 100 ml Wasser verdünnt und zuerst mit 150 ml eines 1:1-Gemisches von Essigsäureäthylester und Äther und dann mit 100 ml Chloroform extrahiert. Der nach Eindampfen der vereinigten Extrakte erhaltene trockene Rückstand wird in 100 ml Äther gelöst und mit 20 ml äthanolischer Chlorwasserstoffsäure behandelt. Das als Niederschlag erhaltene Salz wird abgetrennt, zuerst aus 50 ml eines Gemisches von Acetonitril/Essigsäureäthylester (1:1) und dann aus 30 ml Äthanol/Äther-Gemisch (1:1) umkristallisiert. Man erhält das 5-[5-(N,N-Dimethylcarbamoyl)-pentyl]-imidazo[-1,5-a] pyridin-hydrochlorid, welches bei 166–171° schmilzt.

Beispiel 13:

Man suspendiert 1,0 g 5-(5-Carboxypentyl)-imidazo[1,5-a]pyridin in 5 ml Tetrahydrofuran. Die Suspension wird unter Rühren bei Zimmertemperatur zuerst mit 2,35 g Trimethylborat und dann langsam mit 1,0 ml (äquivalent mit 0,01 Mol) Boran-methylsulfid-Komplex versetzt. Das Reaktionsgemisch wird 2 Stunden bei Rückflusstemperatur erhitzt, abgekühlt und durch Zugabe von 2,6 ml Methanol, 9,5 ml Wasser und 2 ml 50%iger wässeriger Natriumhydroxidlösung gelöscht. Das Gemisch wird 1 Stunde unter Rückfluss gekocht, mit 50 ml Wasser verdünnt und zweimal mit je 75 ml Methylenchlorid extrahiert. Der Methylenchlorid-Extrakt wird zur Trockene eingedampft und der Rückstand mit 4 ml 5-normaler Chlorwasserstoffsäure in 30 ml Äther behandelt. Man erhält das 5-(6-Hydroxyhexyl)-imidazol[1,5-a]pyridin-hydrochlorid, welches bei 174–179° schmilzt.

Beispiel 14:

Eine Lösung von 11,1 g 1-Tetrahydropyranyloxy-8-bromoktan in 15 ml Tetrahydrofuran wird bei –70° zu einer Lösung von 5-(Lithiomethyl)-imidazo[1,5-a]pyridin (hergestellt gemäss Beispiel 1 aus 5 g 5-Methylimidazo[1,5-a]pyridin und 17,7 ml 2,3-normalem n-Butyllithium in Hexan) gegeben. Das Gemisch wird 1 Stunde bei –70° und dann ohne zusätzliche Kühlung über Nacht gerührt. Das Gemisch wird zur Trockene eingedampft, der Rückstand in 50 ml 4-normaler Chlorwasserstoffsäure gelöst, die Lösung 2mal mit je 100 ml Äther gewaschen, mit 75 ml wässeriger Natriumhydroxidlösung basisch gemacht und zweimal mit je 100 ml Methylenchlorid extrahiert. Der Methylenchlorid-Extrakt wird zur Trockene eingedampft. Der Rückstand wird mit ätherischer Chlorwasserstoffsäure in das Hydrochlorid umgewandelt und aus Äthanol/Äther umkristallisiert. Man erhält das 5-(9-Hydroxynonyl)-imidazo[1,5-a]pyridin-hydrochlorid, welches bei 150–153° schmilzt.

Beispiel 15:

a) Eine Lösung von 2,7 g 5-(6-Carboxyhexyl)-imidazo[1,5-a]pyridin in einem Gemisch von 120 ml Äthanol und 30 ml konz. Chlorwasserstoffsäure wird bei 3 Atmosphären in Gegenwart von 1 g 10%igem Palladium-auf-Kohle-Katalysator bis zur Aufnahme von 2 Mol Wasserstoff hydriert. Das Gemisch wird vom Katalysator abfiltriert und zur Trockene eingedampft. Der Rückstand wird aus Isopropanol/Äther umkristallisiert. Man erhält das 5-(6-Carboxyhexyl)-5,6,7,8-tetrahydroimidazo [1,5-a]pyridin-hydrochlorid, welches bei 150–154° schmilzt.

b) In analoger Weise erhält man durch Hydrierung von 5-(5-Carboxypentyl)-imidazo[1,5-a]pyridin das 5-(5-Carboxypentyl)-5,6,7,8-tetrahydroimidazo[1,5-a]pyridin-hydrochlorid, welches bei 146–150° schmilzt.

c) Ausgehend von 5-(4-Carboxybutyl)-imidazo[1,5-a]pyridin erhält man in analoger Weise das 5-(4-Carboxybutyl)-5,6,7,8-tetrahydro-imidazo[1,5-a]pyridin-hydrochlorid, welches bei 120–123° schmilzt.

Beispiel 16:

Eine Lösung von 2,3 g (0,011 Mol) 5-Brom-3,3-dimethylpentansäure [J. Org. Chem. 44, 1258 (1979)] in 20 ml trockenem Tetrahydrofuran wird in einer Stickstoffatmosphäre auf –70° gekühlt und mit 5,05 ml 2,4-molarem n-Butyllithium in Hexan tropfenweise versetzt. Nach dem Abschluss der Zugabe wird eine Lösung von 5-(Li-

thiomethyl)-imidazo[1,5-a]pyridin in Hexan (hergestellt aus 1,32 g 5-Methyl-imidazo[1,5-a]pyridin und 5,05 ml 2,4-normalem n-Butyllithium in Hexan) auf einmal zugegeben. Das Gemisch wird über Nacht bei Zimmertemperatur gerührt.

Das Reaktionsgemisch wird mit 50 ml Wasser verdünnt, mit 10 g Natriumcarbonat versetzt und die basische Lösung wird dreimal mit je 75 ml Chloroform extrahiert. Die wässerige Phase wird mit 12-normaler Chlorwasserstoffsäure auf den pH-Wert 2 angesäuert und dreimal mit je 100 ml Äther extrahiert. Schliesslich wird die wässerige Phase mit verdünnter Natriumhydroxidlösung auf den pH-Wert 5 eingestellt und mit 200 ml eines Essigsäureäthylester/Äther-Gemisches (1:1) extrahiert. Die Extrakte werden getrocknet und eingedampft, wobei man ein gelbes Öl erhält. Dieses wird aus 50 ml eines Äthanol/Äther-Gemisches (1:1) umkristallisiert. Man erhält das 5-(5-Carboxy-4,4-dimethylpentyl)-imidazo[1,5-a]pyridin, welches bei 124–129° schmilzt.

Beispiel 17:

Man gibt 1,9 g Jodkristalle zu einer stark gerührten Lösung von 1,16 g 5-(5-Carboxypentyl)-imidazo[1,5-a]pyridin und 1,68 g Natriumcarbonat in 10 ml Wasser and 1 ml Äthanol. Zur Auflösung des Grossteils von Jod gibt man weitere 4 ml Äthanol dazu und rührt das Gemisch weitere 45 Minuten. Das Reaktionsgemisch wird mit 12 ml Wasser verdünnt und zweimal beim pH-Wert 8 (falls nötig unter Zugabe von Natriumhydrogencarbonat) mit Methylenchlorid extrahiert. Die wässerige Phase wird im Vakuum konzentriert mit Aktivkohle gereinigt und mit 2-normaler Chlorwasserstoffsäure auf den pH-Wert 4,5 eingestellt. Der Niederschlag wird abgetrennt, getrocknet und aus Methanol/Äther umkristallisiert. Man erhält das 1-Jod-5-(5-carboxypentyl)-imidazo[1,5-a]pyridin, welches bei 163–165° schmilzt.

Beispiel 18:

Herstellung von 10'000 Tabletten mit einem Gehalt von je 10 mg der aktiven Substanz des Beispiels 2:

Bestandteile:

| | |
|---|---|
| 5-(5-Carboxypentyl)-imidazo[1,5-a]pyridin | 100 g |
| Milchzucker | 1157 g |
| Maisstärke | 75 g |
| Polyäthylenglykol 6000 | 75 g |
| Talkpulver | 5 g |
| Magnesiumstearat | 18 g |
| gereinigtes Wasser | q.s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 19:

Herstellung von 10'000 Kapseln mit einem Gehalt von je 25 mg der aktiven Substanz des Beispiels 3b:

Bestandteile:

| | |
|---|---|
| 1-(6-Carboxyhexyl)-imidazo[1,5-a]pyridin | 250 g |
| Milchzucker | 1800 g |
| Talkpulver | 100 g |

Verfahren:

Sämtliche pulverigen Bestandteile werden mit einem Sieb von 0,6 mm Maschenweite gesiebt. Dann wird der Wirkstoff zuerst mit Talk und nachher mit Milchzucker in einem geeigneten Mischer homogenisiert. Kapseln Nr. 3 werden mit je 215 mg der erhaltenen Mischung in einer Füllmaschine abgefüllt.

In analoger Weise werden auch Tabletten und harte Gelatine-Kapseln unter Verwendung von Produkten der anderen Beispiele, hergestellt.

Beispiel 20:

Eine Lösung von 5-Methylimidazo[1,5-a]pyridin (4,0 g) und Tetramethyl-äthylendiamin (4,9 g) in 100 ml Tetrahydrofuran wird unter Stickstoff auf 0° gekühlt und mit 26,5 ml 1,6-normaler n-Butyllithium in Hexan tropfenweise versetzt, wobei man die Temperatur unter 2° hält. Diese Lösung wird nach 30 Minuten, in einer Stickstoffatmosphäre, innerhalb 45 Minuten zu einer eiskalten Lösung von 5-Brom-valeronitril (4,86 g) in 80 ml Tetrahydrofuran gegeben. Das Lösungsmittel wird nach 15 Minuten abgedampft und der Rückstand zwischen Wasser und Essigsäureäthylester verteilt. Die organische Phase wird mit 2-normaler Chlorwasserstoffsäure (3×15 ml) extrahiert. Die wässerige Phase wird mit 50%iger Natriumhydroxidlösung auf den pH-Wert 10 eingestellt. Man extrahiert mit Essigsäureäthylester (2×75 ml), trocknet mit Magnesiumsulfat, dampft ein und chromatographiert (Silicagel, Essigsäureäthylester). Man erhält das 5-(5-Cyanpentyl)-imidazo[1,5-a]pyridin.

Beispiel 21:

Eine Lösung von 4 g 5-(4-Äthoxycarbonylbutyl)-3-äthylthio-imidazo[1,5-a]pyridin in 100 ml Äthanol wird mit ungefähr 5 g Raney-Nickel versetzt. Die Lösung wird 18 Stunden unter Rückfluss gekocht. Das Raney-Nickel wird abfiltriert und mit 100 ml Essigsäureäthylester gewaschen. Das Filtrat wird unter vermindertem Druck zur Trockene eingedampft. Man erhält das Produkt als ein schweres Öl. Dieses wird durch Säulenchromatographie (Silicagel) gereinigt und mit einem (1:3)-Gemisch von Äther-Hexan eluiert. Das Lösungsmittel wird unter vermindertem

Druck eingedampft. Man erhält das 5-(4-äthoxycarbonyl-butyl)-imidazo[1,5-a]pyridin als gelbes Öl. NMR ($CDCl_3$) 1,25 (t, 3H), 4,15 (q, 2H), 8,1 (s, 1H).

Der Ausgangsstoff wird wie folgt hergestellt:

Man löst 17,8 g 3-Äthylthio-imidazo[1,5-a]pyridin in 200 ml trockenem Tetrahydrofuran und kühlt die Lösung auf -70°. Innerhalb 15 Minuten gibt man tropfenweise, unter Rühren, 80 ml von 1,6-molarem n-Butyl-lithium in Hexan dazu. Nach beendeter Zugabe wird das Reaktionsgemisch weitere 30 Minuten bei -70° gerührt. Das Gemisch wird mit einer Lösung von 20 g 4-Brompentansäure-äthylester in 75 ml Tetrahydrofuran tropfenweise versetzt. Das Reaktionsgemisch lässt man sich auf -10° erwärmen, hält es 30 Minuten bei dieser Temperatur und nachfolgend 1 Stunde bei Raumtemperatur. Das Reaktionsgemisch wird mit 400 ml Diäthyläther und 400 ml 4-normaler Chlorwasserstoffsäure versetzt. Die wässerige Phase wird abgetrennt und die ätherische Schicht mit Wasser gewaschen. Die vereinigten wässerigen Extrakte werden mit Ammoniumhydroxid basisch gemacht und dreimal mit je 200 ml Äther extrahiert. Der Ätherextrakt wird über wasserfreiem Magnesiumsulfat getrocknet und das Lösungsmittel bei vermindertem Druck abgedampft. Als Rohprodukt erhält man ein schweres Öl. Dieses wird auf einer Silicagelsäule chromatographiert und mit einem (4:1)-Gemisch von Pentan-Diäthyläther eluiert. Das Lösungsmittel wird abgedampft und das Produkt destilliert. Man erhält das 3-Äthylthio-5-(4-äthoxycarbonyl-butyl)-imidazo[1,5-a]pyridin, welches bei 170°/0,3 mm Hg siedet. NMR ($CDCl_3$) 1,25 (t, 3H), 1,30 (t, 3H), 3,15 (q, 2H), 4,15 (q, 2H).

Beispiel 22:

Eine Lösung von 3 g 5-[5-Äthoxycarbonyl-5-(phenylsulfinyl)-pentyl]-imidazo[1,5-a]pyridin in 50 ml Xylol wird unter Stickstoff 30 Minuten unter Rückfluss gekocht. Das Xylol wird dann unter vermindertem Druck abgedampft, der Rückstand in 15 ml Diäthyläther gelöst und auf Silicagel chromatographiert. Das Produkt wird mit einem (2:1)-Gemisch von Diäthyläther und Essigsäureäthylester eluiert. Nach Abdampfen des Lösungsmittels erhält man das 5-(5-Äthoxycarbonylpent-4-enyl)-imidazo[1,5-a]pyridin als ein Öl. NMR ($CDCl_3$) 1,29 (t, 3H), 4,25 (q, 2H), 5,88 (d, 1H).

Der Ausgangsstoff wird wie folgt hergestellt:

Eine eisgekühlte, magnetisch gerührte Aufschwemmung von 0,96 g Natriumhydrid in 50 ml Dimethylformamid wird innerhalb 15 Minuten mit 3,92 g 2-(Phenylthio)-essigsäureäthylester tropfenweise versetzt. Die Suspension wird bei Zimmertemperatur 2 Stunden gerührt und dann mit einem Eisbad auf 5° gekühlt. Diese Suspension wird innerhalb 1 Stunde mit 4,16 g 5-(4-Chlorbutyl)-imidazo[1,5-a]pyridin tropfenweise versetzt. Dann wird das Gemisch mit 3,2 g Natriumjodid versetzt und über Nacht bei Zimmertemperatur gerührt.

Das Reaktionsgemisch wird in 150 ml Eiswasser gegossen und dreimal mit je 100 ml eines (1:1)-Gemisches von Diäthyläther und Essigsäureäthylester extrahiert. Die organische Phase wird zweimal mit je 100 ml gesättigter wässeriger Natriumchloridlösung gewaschen und dann dreimal mit je 50 ml 1-normaler Chlorwasserstoffsäure extrahiert. Die saueren wässerigen Extrakte werden vereinigt, mit Ammoniumhydroxid basisch gemacht und dreimal mit je 150 ml eines 1:1-Gemisches von Diäthyläther und Essigsäureäthylester extrahiert. Die organischen Extrakte werden über wasserfreiem Magnesiumsulfat getrocknet, filtriert und unter vermindertem Druck eingedampft. Man erhält ein Öl, welches durch Säulenchromatographie auf Silicagel gereinigt und mit Diäthyläther eluiert wird. Nach Abdampfen des Lösungsmittels erhält man das 5-[5-Äthoxycarbonyl-5- (phenylthio)-pentyl]-imidazo [1,5-a]pyridin als ein schweres Öl. NMR ($CDCl_3$) 3,3-3,8 (1H); IR 1720 $cm^{-1}$.

Eine Lösung von 3,8 g 5-[5-Äthoxycarbonyl-5-(phenylthio)-pentyl]-imidazo[1,5-a]pyridin in 100 ml Methanol wird mit 2,8 g Natrium-metaperjodat versetzt und das Gemisch 18 Stunden bei Raumtemperatur gerührt. Das Lösungsmittel wird unter vermindertem Druck abgedampft und der Rückstand in 150 ml Wasser aufgenommen. Man extrahiert dreimal mit je 100 ml Essigsäureäthylester. Die organische Phase wird zweimal mit je 50 ml 1-normaler Chlorwasserstoffsäure extrahiert, der wässerige Extrakt mit Ammoniumhydroxid basisch gemacht und wieder mit 2×100 ml Essigsäureäthylester extrahiert. Die vereinigten Essigsäureäthylester-Extrakte werden über wasserfreiem Magnesiumsulfat getrocknet, filtriert und unter vermindertem Druck eingedampft. Man erhält ein Öl, welches durch Säulenchromatographie auf Silicagel gereinigt und mit einem (1:1)-Gemisch von Essigsäureäthylester und Diäthyläther eluiert wird. Nach Eindampfen erhält man das 5-[5-Äthoxycarbonyl-5-(phenylsulfinyl)-pentyl]-imidazo [1,5-a]pyridin als ein Öl. IR 1720 $cm^{-1}$, 1040 $cm^{-1}$.

Beispiel 23:

Eine Lösung von 300 mg 5-(5-Äthoxycarbonylpent-4-enyl)-imidazo[1,5-a]pyridin in 20 ml Methanol wird mit 5 ml 1-normaler Natriumhydroxidlösung versetzt und das Gemisch 18 Stunden bei Raumtemperatur gerührt. Das Methanol wird unter vermindertem Druck abgedampft und der wässerige Rückstand mit weiteren 5 ml Wasser versetzt. Das Gemisch wird dreimal mit je 5 ml Essigsäureäthylester extrahiert. Die basische wässerige Schicht wird dann auf den pH-Wert 5 eingestellt und dreimal mit je 5 ml Essigsäureäthylester extrahiert. Diese Extrakte werden über wasserfreiem Natriumsulfat getrocknet, filtriert und unter vermindertem Druck eingedampft. Man erhält das 5-(5-Carboxypent-4-enyl)-imidazo [1,5-a]pyridin, welches bei 142-144° schmilzt.

Beispiel 24:

Zu einer Lösung von 2,75 g 5-(5-Formylpentyl)-imidazo[1,5-a]pyridin in 180 ml Chloroform gibt man 6,5 Carbäthoxymethylen-triphenylphosphoran und rührt das Gemisch 18 Stunden bei Raumtemperatur. Das Lösungsmittel wird dann unter vermindertem Druck abgedampft. Man erhält das 5-(7-Äthoxycarbonyl-hept-6-enyl)-imidazo [1,5-a]pyridin als ein Öl.

Der Ausgangsstoff wird wie folgt hergestellt:

Eine auf -60° gekühlte Lösung von 4,9 g 5-(5-Methoxycarbonyl-pentyl)-imidazo[1,5-a]-pyridin (welches durch Veresterung des im Beispiel 2 hergestellten 5-(5-Carboxymethyl)-imidazo [1,5-a]pyridins mit Diazomethan in Methylenchlorid erhalten wird) in 140 ml Methylenchlorid wird innerhalb 20 Minuten mit 40 ml einer 1,75-molaren Lösung von Di-isobutyl-aluminiumhydrid in Hexan tropfenweise versetzt. Dann wird das Reaktionsgemisch 20 Minuten bei -60° gerührt und nachfolgend mit 10 ml Methanol und 100 ml Wasser zur Unterbrechung der Reaktion versetzt. Das Gemisch wird bei Raumtemperatur 15 Minuten gerührt, die Methylenchlorid-Schicht abgetrennt und das Lösungsmittel bei vermindertem Druck abgedampft. Man erhält das 5-(5-Formylpentyl)-imidazo[1,5-a]pyridin als ein Öl. NMR ($CDCl_3$) 9,7 (m, 1H); IR (Methylenchlorid) 1710 $cm^{-1}$.

Beispiel 25:

Eine Lösung von 2,8 g 5-(7-Äthoxycarbonyl-hept-6-enyl)-imidazo[1,5-a]pyridin in 30 ml Methanol wird mit 15 ml 1-normaler Natriumhydroxidlösung versetzt und das Gemisch bei Raumtemperatur 3 Stunden gerührt. Das Methanol wird unter vermindertem Druck abgedampft, der Rückstand mit 30 ml Wasser verdünnt und die Lösung mit 1-normaler Chlorwasserstoffsäure auf den pH-Wert 7 eingestellt. Die Lösung wird mit 2×50 ml Essigsäureäthylester extrahiert. Die vereinigten Extrakte werden über wasserfreiem Magnesiumsulfat getrocknet, filtriert und das Lösungsmittel unter vermindertem Druck abgedampft. Man erhält das 5-(7-Carboxyhept-6-enyl)-imidazo[1,5-a]pyridin, welches bei 110–111° schmilzt.

Beispiel 26:

Eine Lösung von 150 mg 5-(5-Carboxypent-4-enyl)-imidazo[1,5-a]pyridin in 7 ml Methanol wird mit 100 mg 10%igem Palladium-auf-Kohle-Katalysator versetzt. Das Reaktionsgemisch wird 3 Stunden bei atmosphärischem Druck hydriert. Der Katalysator wird abfiltriert und das Lösungsmittel unter vermindertem Druck abgedampft. Man erhält das 5-(5-Carboxypentyl)-imidazo-[1,5-a]pyridin, welches bei 144–147° schmilzt und mit dem Produkt des Beispiels 2 identisch ist.

Beispiel 27:

Eine Lösung von 180 mg 5-(7-Carboxyhept-6-enyl)-imidazo[1,5-a]pyridin in 30 ml Methanol wird mit 200 mg 10%igem Palladium-auf-Kohle-Katalysator versetzt und das Gemisch 3 Stunden bei atmosphärischem Druck hydriert. Der Katalysator wird abfiltriert und das Lösungsmittel unter vermindertem Druck abgedampft. Man erhält das Produkt, welches bei 69–71° schmilzt und aus dem Gemisch von 5-(7-Carboxyheptyl)-imidazo[1,5-a]pyridin (Verbindung des Beispiels 3c) und von 5-(7-Carboxyheptyl)-5,6,7,8-tetrahydroimidazo[1,5-a]pyridin besteht.

Beispiel 28:

Eine Lösung von 0,1 g 2-Aminomethyl-3-(4-methoxycarbonylbutyl)-pyridin in 0,6 ml Ameisensäure wird 18 Stunden bei 90° erhitzt. Das Gemisch wird auf 0° gekühlt, mit gesättigter wässeriger Ammoniumhydroxidlösung basisch gemacht und mit Methylenchlorid (4×10 ml) extrahiert. Die Extrakte werden getrocknet, filtriert und eingedampft. Man erhält das 2-(N-Formylaminomethyl)-3-(4-methoxycarbonylbutyl)-pyridin, welches bei 43–45° schmilzt. Dieses wird in 1 ml Toluol wieder aufgelöst und 17 Stunden bei 90° mit 75 mg Phosphoroxychlorid erhitzt. Das überschüssige Phosphoroxychlorid wird mit Toluol abgedampft, der Rückstand bei 0° mit gesättigter wässeriger Ammoniumhydroxidlösung basisch gemacht, mit Methylenchlorid (4×15 ml) extrahiert und der Extrakt über Natriumsulfat getrocknet. Nach Eindampfen erhält man ein Öl, das nach Chromatographie (Silicagel, Essigsäureäthylester) ein Öl ergibt. Dieses ist das 8-(4-Methoxycarbonylbutyl)-imidazo[1,5-a]pyridin. Rf = 0,29; NMR ($CDCl_3$) 3,70 (s, 3H), 6,50 (d, 2H), 7,43 (s, 1H), 7,83 (t, 1H), 8,22 (s, 1H); IR (Methylenchlorid) 1725 $cm^{-1}$.

Der Ausgangsstoff wird wie folgt hergestellt:

Eine Lösung von 3-Brompyridin (7,9 g), 4-Pentensäure-methylester (7,15 g), Palladiumacetat (0,11 g) und Tri-o-tolylphosphin (0,6 g) in 50 ml Triäthylamin wird 24 Stunden unter Argon unter Rückfluss gekocht und das Lösungsmittel abgedampft. Der Rückstand wird in Methylenchlorid aufgenommen (50 ml) und mit Wasser (2×40 ml) gewaschen. Die organische Phase wird getrocknet und eingedampft. Man erhält das 3-(4-Methoxycarbonylbut-1-enyl)-pyridin als eine farblose Flüssigkeit. NMR ($CDCl_3$) 3,72 (s, 3H), 6,40 (s, 1H); IR (Film) 1725 $cm^{-1}$.

3-(4-Methoxycarbonylbut-1-enyl)-pyridin (9,5 g) wird in 100 ml Methanol mit 0,5 g 5%igem Palladium-auf-Kohle-Katalysator 3,5 Stunden bei 3 Atmosphären hydriert. Nach Filtrierung und Eindampfen erhält man das 3-(4-Methoxycarbonylbutyl)-pyridin als ein Öl. NMR ($CDCl_3$) 3,80 (s, 3H); IR (Methylenchlorid) 1730 $cm^{-1}$.

3-(4-Methoxycarbonylbutyl)-pyridin (10,81 g) wird tropfenweise, wobei man die Temperatur des Reaktionsgemisches zwischen 80-85° hält, mit Peressigsäure (40%, 8,3 ml) versetzt. Nachher lässt man die Temperatur auf 30° sinken und die überschüssige Peressigsäure wird mit wässeriger Natriumsulfitlösung zerstört. Die Essigsäure wird unter vermindertem Druck abgedampft, der Rückstand in Methylenchlorid (50 ml) aufgenommen, filtriert und eingedampft. Der Rückstand, der aus 3-(4-Methoxycarbonylbutyl)-pyridin-N-oxid besteht, wird mit Dimethylsulfat (7,7 g) in 40 ml Toluol bei 90° eine Stunde behandelt und das Lösungsmittel abgedampft. Das 3-(4-Methoxycarbonylbutyl)-1-methoxypyridinium-methylsulfat-Salz wird in 16,7 ml eiskaltem Wasser und 8,3 ml 1-normaler Natriumhydroxidlösung gelöst und mit einer Lösung von Kaliumcyanid (11,21 g) in 16,7 ml eiskaltem Wasser langsam, wobei man die Temperatur bei 0° hält, versetzt. Nach 24 Stunden bei 0°, extrahiert man mit Methylenchlorid (3×30 ml), trocknet den Extrakt über Natriumsulfat und dampft das Lösungsmittel ab. Man erhält ein Gemisch von isomeren Cyanpyridinen, von welchen man das 2-Cyan-3-(4-methoxycarbonylbutyl)-pyridin mit dem Rf-Wert von 0,56 und NMR ($CdCl_3$) 8,82 (m, 1H) und das 2-Cyan-5-(4-methoxycarbonylbutyl)-pyridin mit dem Rf-Wert 0,50 und NMR ($CDCl_3$) 8,72 (s, 1H) durch Chromatographie getrennt werden (Silicagel, Äther-Pentan 3:2).

2-Cyan-3-(4-methoxycarbonylbutyl)-pyridin (2,40 g) wird in 92 ml Methanol, welches 2,4 ml konz. Chlorwasserstoffsäure enthält, gelöst und mit 1,2 g 10%igem Palladium-auf-Kohle-Katalysator 3 Stunden bei atmosphärischem Druck hydriert. Filtrieren, Eindampfen und Umkristallisation aus Äther-Methylenchlorid ergibt das 2-Aminomethyl-3-(4-methoxycarbonylbutyl)-pyridin-hydrochlorid, welches bei 79–81° schmilzt.

Beispiel 29:

Eine Lösung von 8-(4-Methoxycarbonylbutyl)-imidazo[1,5-a]pyridin (30 mg) in 0,3 ml Äthanol und 0,3 ml 1-normaler Natriumhydroxidlösung wird 2 Stunden unter Rückfluss gekocht, abgekühlt, mit 2 ml Wasser verdünnt und mit Essigsäureäthylester (1×5 ml) extrahiert. Die wässerige Phase stellt man auf den pH-Wert 6 ein und extrahiert mit Methylenchlorid (4×10 ml). Die Extrakte werden getrocknet und eingedampft. Man erhält das 8-(4-Carboxybutyl)-imidazo[1,5-a]pyridin, welches bei 195–197° schmilzt.

Beispiel 30:

2-Aminomethyl-5-(4-methoxycarbonylbutyl)-pyridin (0,20 g) wird in 0,6 ml Ameisensäure 18 Stunden auf 90° erhitzt. Das Gemisch wird auf 0° gekühlt, mit gesättigter wässeriger Ammoniumhydroxidlösung basisch gemacht und mit Methylenchlorid (4×15 ml) extrahiert. Die Extrakte werden getrocknet, filtriert und eingedampft. Man erhält das 2-(N-Formylaminomethyl)-5-(4-methoxycarbonylbutyl)-pyridin als ein Öl (IR 1720, und 1675 $cm^{-1}$), welches in 1 ml Toluol gelöst und 18 Stunden bei 90° mit Phosphoroxychlorid (0,166 g) erhitzt wird. Abdampfen vom überschüssigen Phosphoroxychlorid mit Toluol und Zugabe bei 0° von gesättigter wässeriger Ammoniumhydroxidlösung, Extraktion der basischen Lösung mit Methylenchlorid (4×15 ml) und Trocknen des Extraktes über Natriumsulfat, ergibt ein Öl, welches chromatographiert (Silicagel, Essigsäureäthylester) wird. Man erhält das 6-(4-Methoxycarbonylbutyl)-imidazo[1,5-a]pyridin. Rf = 0,26; NMR ($CDCl_3$) 3,58 (s, 3H), 6,45 (d, 1H), 7,25 (d, 1H), 7,38 (s, 1H), 7,62 (s, 1H), 7,94 (s, 1H); IR (Methylenchlorid) 1730 $cm^{-1}$.

Der Ausgangsstoff wird wie folgt hergestellt:

2-Cyan-5-(4-methoxycarbonylbutyl)-pyridin (1,48 g, s. Beispiel 28) wird in 56 ml Methanol, welches 1,5 ml konz. Chlorwasserstoffsäure enthält, gelöst und über 0,75 g 10%igem Palladium-auf-Kohle-Katalysator 18 Stunden bei atmosphärischem Druck hydriert. Das Gemisch wird filtriert, das Filtrat eingedampft, der Rückstand auf 20 g Silicagel chromatographiert und mit 1:1 Methanol-Essigsäureäthylester eluiert. Nach Umkristallisation aus Äther-Methylenchlorid erhält man das 2-Aminomethyl-5-(4-methoxycarbonylbutyl)-pyridin als sein Carbonat, welches bei 79–80° schmilzt. NMR ($CDCl_3$) 3,67 (s, 3H), 4,24 (s, 2H); IR (Methylenchlorid) 1725 $cm^{-1}$.

Beispiel 31:

Eine Lösung von 92 mg 6-(4-Methoxycarbonylbutyl)-imidazol[1,5-a]pyridin in 0,3 ml Äthanol und 0,8 ml 1-normaler Natriumhydroxidlösung wird 2 Stunden unter Rückfluss sanft erhitzt, gekühlt, mit 2 ml Wasser verdünnt und mit Essigsäureäthylester (5 ml) extrahiert. Die wässerige Phase wird auf den pH-Wert 6 eingestellt und mit Chloroform extrahiert. Die Extrakte werden getrocknet und eingedampft. Man erhält das 6-(4-Carboxybutyl)-imidazo[1,5-a]pyridin, welches bei 168–171° schmilzt.

Beispiel 32:

2-(N-Formylaminomethyl)-4-(3-methoxycarbonylpropyl)-pyridin (33 mg) wird in 1 ml Toluol gelöst und mit Phosphoroxychlorid (44 mg) unter Stickstoff 18 Stunden bei 90° erhitzt. Das Lösungsmittel wird abgedampft, der Rückstand in Methylenchlorid suspendiert, auf 0° gekühlt und mit gesättigter wässeriger Ammoniumhydroxidlösung basisch gemacht. Die wässerige Phase wird mit Methylenchlorid (4×15 ml) extrahiert. Die Extrakte werden über Natriumsulfat getrocknet und eingedampft. Man erhält das 7-[3-Methoxycarbonylpropyl)-imidazo[1,5-a]pyridin als ein Öl, welches durch präparative Dünnschichtchromatographie gereinigt wird (Silicagel, 3:1 Essigsäureäthylester-Methanol). NMR ($CDCl_3$) 3,70 (s, 3H), 6,45 (q, 1H), 7,2 (s, 1H), 7,32 (s, 1H), 7,90 (d, 1H), 8,08 (s, 1H); IR (Methylenchlorid) 1730 $cm^{-1}$.

Der Ausgangsstoff wird wie folgt hergestellt:

Kaliumcyanid (11,18 g) und Dibenzo-18-kronen-6-äther (1,0 g) werden in einer Stickstoffatmosphäre zu einer Lösung von 4-(3-Chlorpropyl)-pyridin (6,68 g), (hergestellt aus 4-(3-Hydroxypropyl)-pyridin), in 300 ml trockenem Acetonitril gegeben. Das Gemisch wird 24 Stunden unter Rückfluss gekocht, das Lösungsmittel abgedampft und der Rückstand zwischen Methylenchlorid und Wasser verteilt. Die wässerige Phase wird mit Methylenchlorid (3×100 ml) weiter extrahiert. Die vereinigten Extrakte werden über Natriumsulfat getrocknet, mit Aktivkohle entfärbt

und eingedampft. Man erhält das 4-(3-Cyanpropyl)-pyridin als ein farbloses Öl.

Durch eine Lösung von 4-(3-Cyanpropyl)-pyridin (5,5 g) in Methanol wird unter Eiskühlung Chlorwasserstoff 2 Stunden geleitet und die Lösung mit 100 ml Wasser vorsichtig versetzt. Die Lösung wird 15 Minuten gerührt und das Lösungsmittel abgedampft. Der Rückstand wird mit gesättigter wässeriger Natriumhydrogencarbonatlösung basisch gemacht und mit Methylenchlorid (3×100 ml) extrahiert. Der Extrakt wird über Natriumsulfat getrocknet und eingedampft. Der Rückstand wird in Äther durch 50 g Silicagel filtriert. Man erhält das 4-(3-Methoxycarbonylpropyl)-pyridin als ein Öl. NMR ($CDCl_3$) 3,68 (s, 3H), 7,05–7,25 (m, 2H), 8,45–8,65 (m, 2H); IR: 1725 $cm^{-1}$.

Bei Zimmertemperatur gibt man Peressigsäure (40%, 2,9 ml) zu 4-(3-Methoxycarbonylpropyl)-pyridin (3,20 g). Das Gemisch wird bei 80° eine Stunde erhitzt und wenn der Test für Peroxid negativ ist, die Essigsäure abgedampft. Der Rückstand wird in Methylenchlorid (50 ml) aufgenommen, filtriert und eingedampft. Das erhaltene 4-(3-Methoxy-carbonylpropyl)-pyridin-N-oxid wird mit Dimethylsulfat (2,8 g, 22,2 mMol) in 12 ml Toluol 1 Stunde bei 80° behandelt. Nach Eindampfen des Lösungsmittels erhält man 5,45 g des 4-(3-Methoxycarbonylpropyl)-1-methoxypyridinium-methylsulfats, welches zu einer Lösung von 89,75 g Kaliumcyanid in 20 ml Wasser gegeben wird. Das Reaktionsgemisch wird eine Stunde bei 0° und 3 Stunden bei 25° gerührt und dann mit Methylenchlorid (1×30 ml) extrahiert. Die wässerige Phase wird 24 Stunden stehen gelassen und dann mit Methylenchlorid (1×30 ml) extrahiert. Die vereinigten Extrakte werden über Natriumsulfat getrocknet und eingedampft. Man erhält ein rotes Öl, welches nach Chromatographie auf 70 g Silicagel und Eluierung mit Äther, das 2-Cyan-4-(3-methoxycarbonylpropyl)-pyridin als ein Öl ergibt. NMR ($CDCl_3$) 3,67 (s, 3H), 7,42 (d, 1H), 7,60 (s, 1H), 8,60 (d, 1H); IR (Methylenchlorid) 1725 $cm^{-1}$.

2-Cyan-4-(3-methoxycarbonylpropyl)-pyridin (0,83 g) in 9 ml Methanol wird über 0,4 g 10%igem Palladium-auf-Kohle-Katalysator 3 Stunden bei 3 Atmosphären hydriert. Das Gemisch wird filtriert, das Filtrat eingedampft und der Rückstand durch präparative Dünnschichtchromatographie auf Silicagel mit 1:1 Methanol-Essigsäureäthylester gereinigt. Man erhält das 2-Aminomethyl-4-(3-methoxycarbonylpropyl)-pyridin. Rf = 0,37 (Essigsäureäthylester-Methanol 1:1, 1% Ammoniumhydroxid); NMR ($CDCl_3$) 3,67 (s, 3H), 4,15 (s, 2H).

2-Aminomethyl-4-(3-methoxycarbonylpropyl)-pyridin (0,11 g) in 0,5 ml 97%iger Ameisensäure wird 18 Stunden bei 90° erhitzt. Das Reaktionsgemisch wird auf Zimmertemperatur gekühlt, mit Ammoniumhydroxidlösung basisch gemacht und mit Methylenchlorid (4×20 ml) extrahiert. Die organischen Extrakte werden über Natriumsulfat getrocknet und eingedampft. Man erhält das 2-(N-Formylaminomethyl)-4-(3-methoxycarbonylpropyl)-pyridin. IR (Methylenchlorid) 1735, 1685 $cm^{-1}$.

Beispiel 33:

7-(3-Methoxycarbonylpropyl-3-imidazo [1,5-a] pyridin (Beispiel 32, 8,0 mg) wird in 0,3 ml Methanol gelöst und mit 0,1 ml 1-normaler Natriumhydroxidlösung versetzt. Das Gemisch wird 5 Stunden bei 25° gerührt, eingedampft und der Rückstand in 5 ml Wasser gelöst. Die wässerige Lösung wird mit 2 ml Essigsäureäthylester gewaschen, mit 2-normaler Schwefelsäure auf den pH-Wert 6 eingestellt und mit Methylenchlorid (3×5 ml) extrahiert. Die organischen Extrakte werden über Natriumsulfat/Magnesiumsulfat getrocknet und eingedampft. Man erhält das 7-(3-Carboxypropyl)-imidazo[1,5-a]pyridin; IR ($CHCl_3$) 1720 $cm^{-1}$.

Beispiel 34:

Eine Lösung von 7-[4,4-(Bis-methoxycarbonyl)-butyl]-imidazo[1,5-a]pyridin (65 mg) in 0,8 ml 1-normaler Natriumhydroxydlösung und 0,5 ml Äthanol wird 2 Stunden unter Rückfluss erhitzt. Das Lösungsmittel wird abgedampft und der Rückstand mit 0,8 ml 1-normaler Chlorwasserstoffsäure versetzt. Das Wasser wird abgedampft, der Rückstand in 3 ml Xylol gelöst und 4 Stunden auf 137° erhitzt. Das Xylol wird abgedampft und durch 2 ml 1-normaler Natriumhydroxidlösung ersetzt. Die wässerige Phase wird mit Essigsäureäthylester (5 ml) extrahiert. Ansäuern auf den pH-Wert 6 und Re-extraktion mit Chloroform (3×15 ml) und Eindampfen ergibt das 7-(4-Carboxybutyl)-imidazo[1,5-a]pyridin, welches bei 158–161° schmilzt.

Der Ausgangsstoff wird wie folgt hergestellt.

Gemäss den vorher beschriebenen Verfahren (z.B. Beispiele 28 und 32) wird das 4-(3-Chlorpropyl)-pyridin in das 4-(3-Chlorpropyl)-2-cyanpyridin umgewandelt. NMR ($CDCl_3$) 3,56 (t, 2H), 7,40 (d, 1H), 7,57 (s, 1H), 8,60 (d, 1H).

Eine Lösung von Boran-dimethylsulfid (0,83 ml, 7,7 mMol) in 7 ml Tetrahydrofuran wird langsam zu einer unter Rückfluss kochenden Lösung von 4-(3-Chlorpropyl)-2-cyanpyridin (1,24 g, 6,9 mMol) in 7 ml Tetrahydrofuran gegeben, wobei gleichzeitig Dimethylsulfid abdestilliert wird. Nach der abgeschlossenen Zugabe wird das Gemisch 15 Minuten unter Rückfluss gekocht, auf 30° gekühlt und mit 6-normaler Chlorwasserstoffsäure versetzt. Nach dem Aufhören der Wasserstoffentwicklung wird das Gemisch 30 Minuten unter Rückfluss gekocht, auf 0° gekühlt, mit festem Natriumcarbonat gesättigt und mit Methylenchlorid (4×50 ml) extrahiert. Die organischen Extrakte werden über Natriumsulfat getrocknet und eingedampft. Man erhält ein Öl, welches durch 10 g Silicagel (1:1 Essigsäureäthylester-Methanol) filtriert wird. Man erhält das 2-Aminomethyl-4-(3-chlorpropyl)-pyridin als ein gelbes Öl, NMR ($CDCl_3$) 3,55 (t, 2H), 4,20 (s, 2H).

Eine Lösung von 2-Aminomethyl-4-(3-chlorpropyl)-pyridin (0,47 g) in 1 ml Ameisensäure wird

18 Stunden bei 90° erhitzt, auf 0° gekühlt und mit gesättigter wässeriger Ammoniumhydroxidlösung basisch gemacht. Extraktion mit Methylenchlorid (4×10 ml), Trocknen über Magnesiumsulfat und Eindampfen ergibt das 2-(N-Formylaminomethyl)-4-(3-chlorpropyl)-pyridin (IR 1674 $cm^{-1}$). Dieses wird in Phosphoroxychlorid (0,75 g) 15 Stunden bei 90° erhitzt. Das überschüssige Phosphoroxychlorid wird mit Toluol abgedampft, der Rückstand in Methylenchlorid (15 ml) suspendiert, auf 0° gekühlt und mit gesättigtem Ammoniumhydroxid basisch gemacht. Extraktion mit Methylenchlorid (4×15 ml), Trocknen über Natriumsulfat und Dünnschichtchromatographie (Silicagel, Essigsäureäthylester) des Rückstands ergibt das 7-(3-Chlorpropyl)-imidazo[1,5-a]pyridin (Rf = 0,24, Essigsäureäthylester) als ein gummiartiges Material. NMR ($CDCl_3$) 3,58 (t, 2H), 6,42 (q, 1H), 7,21 (s, 1H), 7,32 (s, 1H), 7,88 (d, 1H), 8,07 (s, 1H).

Eine Lösung von 7-(3-Chlorpropyl)-imidazo[1,5-a]pyridin (50 mg), Malonsäure-dimethylester (0,14 g) und Kaliumcarbonat (14 mg) in 2 ml Dimethylformamid wird unter Stickstoff 9 Stunden zwischen 80 und 90° erhitzt. Das Lösungsmittel wird eingedampft, der Rückstand in 10 ml Wasser aufgenommen und mit Essigsäureäthylester (2×10 ml) extrahiert. Die organischen Extrakte werden mit 2-normaler Chlorwasserstoffsäure (2×10 ml) gewaschen. Die wässerigen Extrakte werden mit festem Natriumhydrogencarbonat basisch gemacht, mit Methylenchlorid (3×10 ml) extrahiert, über Natriumsulfat getrocknet und eingedampft. Man erhält das 7-[4,4-(Bis-methoxycarbonyl)-butyl]-imidazo-[1,5-a]pyridin. NMR ($CDCl_3$) 3,40 (s, 6H), 6,06 (d, 1H); IR (Methylenchlorid) 1725 $cm^{-1}$.

Beispiel 35:

Eine Lösung von 5-[5,5-(Bis-äthoxycarbonyl)-pentyl]-imidazo[1,5-a]pyridin (0,60 g) in 6,5 ml 1-normaler Natriumhydroxidlösung und 4 ml Methanol wird 2 Stunden unter Rückfluss gekocht. Das Lösungsmittel wird abgedampft und 6,5 ml 1-normaler Chlorwasserstoffsäure zugegeben. Das Wasser wird dann abgedampft und das erhaltene 5-[5,5-(Bis-carboxy)-pentyl]-imidazo-[1,5-a]pyridin in 25 ml Xylol 4 Stunden bei 137° erhitzt. Das Xylol wird durch 16 ml 1-normaler Natriumhydroxidlösung ersetzt. Extraktion der wässerigen Phase mit Essigsäureäthylester (15 ml), Ansäuern auf den pH-Wert 6, Re-extraktion mit Chloroform (3×40 ml), Trocknen über Magnesiumsulfat und Eindampfen ergibt das 5-(5-Carboxypentyl)-imidazo[1,5-a]pyridin, welches bei 146–147° schmilzt. (Verbindung des Beispiels 2).

Der Ausgangsstoff wird wie folgt hergestellt:

Eine Lösung von 5-(4-Chlorbutyl)-imidazo[1,5-a]pyridin (0,42 g), Malonsäure-diäthylester (1,34 g) und Kaliumcarbonat (1,15 g) in 20 ml Dimethylformamid wird unter Stickstoff 10 Stunden zwischen 80 und 90° erhitzt. Das Lösungsmittel wird abgedampft und der Rückstand in 50 ml Wasser aufgenommen. Die wässerige Phase wird extrahiert mit Essigsäureäthylester (3×40 ml). Die Extrakte werden mit 2-normaler Chlorwasserstoffsäure (3×10 ml) gewaschen. Die wässerige Phase wird mit festem Natriumcarbonat basisch gemacht, mit Methylenchlorid (3×20 ml) extrahiert, die Extrakte über Natriumsulfat getrocknet und eingedampft. Man erhält das 5-[5,5-Bis-äthoxycarbonyl)-pentyl]-imidazo[1,5-a]pyridin, welches bei 59–61° schmilzt.

Das als Ausgangsstoff verwendete 5-(4-Chlorbutyl)-imidazo[1,5-a]pyridin wird gemäss dem im Beispiel 4 für die Herstellung des Ausgangsstoffes beschriebenen Verfahren, unter Verwendung von 1-Brom-3-chlorpropan als Reagens anstelle vom 1-Brom-4-chlorbutan, hergestellt.

Beispiel 36:

Pyridinium-dichromat (0,94 g) wird in festem Zustand zu einer Lösung von 5-(6-Hydroxyhexyl)-imidazo[1,5-a]pyridin (123 mg) in 10 ml N,N-Dimethylformamid unter Stickstoff bei 25° gegeben. Die Lösung wird 6 Stunden gerührt, in 150 ml Wasser gegossen und mit Methylenchlorid (5 × 20 ml) extrahiert. Die organischen Extrakte werden mit 1-normaler Natriumhydroxidlösung gewaschen. Die wässerige Phase wird auf den pH-Wert 6 angesäuert, mit Methylenchlorid extrahiert, der Extrakt über Natriumsulfat/Magnesiumsulfat getrocknet und eingedampft. Man erhält das 5-(5-Carboxypentyl)-imidazo[1,5-a]pyridin des Beispiels 2. F. 145–146°.

Beispiel 37:

5-Methylimidazo[1,5-a]pyridin [J. Org. Chem. 40, 1210 (1975), 424,7 g] wird in einen 12-Liter-Kolben, der mit einem mechanischen Rührer und Thermometer ausgerüstet und mit Stickstoff gefüllt ist, gegeben. In den Kolben gibt man trockenes Tetrahydrofuran (3000 ml) und kühlt die erhaltene Lösung in einem Trockeneis/Aceton-Bad auf -65° ab. Die Lösung wird mit n-Butyllithium (1,0 Mol, 2,4-normal in Hexan) in einer Stickstoffatmosphäre auf einmal versetzt. Die Temperatur steigt auf -32°. Das Gemisch wird wieder auf -50° gekühlt und in gleicher Weise mit einem weiteren Mol n-Butyllithium versetzt. Die Temperatur steigt wieder und das Gemisch wird nach Kühlung auf -50° mit einem dritten Mol n-Butyllithium versetzt. Das Reaktionsgemisch wird 20 Minuten gerührt, wobei die Temperatur auf -65° fällt. Die gerührte Lösung wird dann so schnell wie möglich mit einer kalten (-67°) Lösung von 5-Brom-1,1,1-triäthoxypentan (606,9 g) in 500 ml Tetrahydrofuran versetzt, wobei die Temperatur auf -25° steigt. Das Reaktionsgemisch wird dann auf -15° erwärmt, 2 Stunden gerührt, mit Essigsäure (50 ml) versetzt und der Hauptteil des Lösungsmittels im Vakuum entfernt. Der Rückstand wird in 2000 ml Äthyläther aufgenommen, mit Essigsäure (100 ml) und 12-normaler Chlorwasserstoffsäure (100 ml) versetzt und auf 0° gekühlt. Das Gemisch wird nach 15 bis 20 Minuten mit eiskaltem, 7,5-normalem Ammoniumhydroxid (1000 ml) versetzt. Die organische Phase wird abgetrennt und die wässerige mit Äthyläther (500 ml)

gewaschen. Der pH-Wert der wässerigen Schicht wird mit Ammoniumhydroxid auf 9 eingestellt und wieder mit Äthyläther (500 ml) extrahiert. Die vereinigten Ätherextrakte werden mit verdünnter Natriumchloridlösung gewaschen, und der pH-Wert wird mit Kaliumhydroxid auf 13–14 eingestellt. Der Ätherextrakt wird mit Aktivkohle und Magnesiumsulfat behandelt. Das Gemisch wird filtriert und eingedampft. Man erhält ein dunkles Öl, welches bei 2 mm Hg getrocknet wird. Das Öl wird unter Hochvakuum destilliert. Man erhält das 5-(5-Äthoxycarbonylpentyl)-imidazo[1,5-a]pyridin des Beispiels 1, welches bei 220°/0,2 mmHg siedet.

Das als Ausgangsstoff verwendete 5-Brom-1,1,1-triäthoxypentan wird wie folgt hergestellt:

5-Bromvaleronitril (1200 g) wird unter Stickstoff in einen 5-Liter-Dreihalskolben gegeben. Der komplette Reaktionskolben wird dann in ein Eisbad gestellt und es werden langsam 287 g Chlorwasserstoffgas eingeleitet. Das Reaktionsgemisch wird dann mit Äthyläther (3200 ml) verdünnt und über Nacht bei 4° gerührt. Die erhaltene Suspension wird in einem Trockeneis/Aceton-Bad auf -30° gekühlt. Das erhaltene feste Material wird abgetrennt, mit Äthyläther gewaschen und in einem Vakuumexsiccator über Kaliumhydroxid und Phosphorpentoxid 3 Tage getrocknet. Man erhält das 5-Bromimidovaleriansäure-äthylesterhydrochlorid, welches im nächsten Schritt ohne weitere Reinigung verwendet wird.

5-Bromimidovaleriansäure-äthylester-hydrochlorid (556 g) wird unter Stickstoff in einen 12-Liter-Kolben, der mit einem mechanischen Rührer ausgerüstet ist, gegeben. Nach Zugabe von wasserfreiem Äthanol (836 g) wird das Reaktionsgemisch 2 Stunden bei Zimmertemperatur gerührt, wobei man eine klare Lösung erhält. Man gibt Äthyläther (3700 ml) in den Kolben und rührt das Gemisch bei Raumtemperatur 3 Tage. Die Lösung wird auf -30° gekühlt und das erhaltene Ammoniumchlorid abfiltriert. Das Filtrat wird in einem Rotationsverdampfer unter Vakuum zur Trockene eingedampft. Der Rückstand wird im Hochvakuum (0,2 mmHg) destilliert, wobei man eine 12-cm-Fraktioniersäule verwendet. Die bei ungefähr 71–82° destillierende Hauptfraktion wird aufgefangen und mit einer 46-cm-Säule redestilliert. Man erhält das 5-Brom-1,1,1-triäthoxypentan, welches bei 60–62°/0,2 mmHg siedet.

Das 5-(5-Äthoxycarbonylpentyl)-imidazo[1,5a]-pyridin kann auch ausgehend vom 2-(N-Formylaminomethyl)-6-(5-äthoxycarbonylpentyl)-pyridin, gemäss dem in den Beispielen 28, 30 und 32 beschriebenen Ringschluss-Verfahren hergestellt werden.

Beispiel 38:

5-(5-Äthoxycarbonylpentyl)-imidazo[1,5-a]pyridin (1091 g) wird unter Stickstoff in einen 12-Liter-Rundkolben gegeben und unter Rühren mit Äthylalkohol (95%, 420 ml) versetzt. Unter Rühren gibt man dann 2-normale Natriumhydroxidlösung (2100 ml) portionenweise dazu. Nach der Zugabe wird das Gemisch 20 Minuten auf 70° erhitzt. Man erhält eine Lösung, die 2 Stunden weiter erhitzt wird. Man gibt eine weitere Menge Natriumhydroxid (50%ige Lösung, 21 ml) dazu und setzt das Erwärmen 40 Minuten fort. Das Reaktionsgemisch wird gekühlt, mit 12-normaler Chlorwasserstoffsäure (30 ml) versetzt und der Äthylalkohol unter vermindertem Druck teilweise abgedampft. Die erhaltene Lösung wird mit Äthyläther (1700 ml) gewaschen, mit Aktivkohle entfärbt, filtriert und mit Essigsäure angesäuert. Das über Nacht bei 4° kristallisierte Produkt wird abgetrennt, zuerst mit Wasser, dann mit Äthyläther (1000 ml) gewaschen und getrocknet. Man erhält das 5-(5-Carboxypentyl)-imidazol[1,5-a]pyridin, welches bei 146–148° schmilzt und mit dem Produkt des Beispiels 2 identisch ist.

Beispiel 39:

Die folgenden Verbindungen werden analog zu den in den vorhergehenden Beispielen beschriebenen Verfahren hergestellt:

a) 5-(4-Äthoxycarbonyl-but-3-enyl)-imidazo-[1,5-a]pyridin durch Kondensation von 5-Methylimidazo[1,5-a]pyridin mit 4-Bromcrotonsäureäthylester;

b) 5-(9-Hydroxynon-7-ynyl)-imidazo[1,5-a]pyridin durch Kondensation von 1-Tetrahydropyranyloxy-8-bromoct-6-yn mit 5-Methyl-imidazo-[1,5-a]pyridin und nachfolgende Hydrolyse.

Beispiel 40:

Eine Lösung von 5-(6-Oxoheptyl)-imidazo[1,5-a]pyridin (0,35 g) in 10 ml Dioxan wird langsam zu einer stark gerührten wässerigen Lösung (3 ml) von Natrium-hypobromit (5,2 mMol) bei 22–25° (Eisbad-Kühlung, falls notwendig) gegeben. Nach 3 Stunden wird das überschüssige Natriumhypobromit mit Natriumbisulfit zerstört und das Lösungsmittel abgedampft. Der Rückstand wird in 10 ml, 0,5-normaler Natriumhydroxidlösung gelöst, mit Äther (2×5 ml) extrahiert und mit konz. Schwefelsäure auf den pH-Wert 6 eingestellt. Nach Extraktion mit Methylenchlorid (3×10 ml), Trocknen über Natriumsulfat/Magnesiumsulfat und Eindampfen erhält man das 5-(5-Carboxypentyl)-imidazo[1,5-a]pyridin des Beispiels 2.

Der Ausgangsstoff wird durch Behandlung von 5-(4-Chlorbutyl)-imidazo[1,5-a]pyridin mit Acetessigsäure-äthylester in Gegenwart von Natriumhydrid und nachfolgende Hydrolyse mit verdünnter Natriumhydroxidlösung hergestellt.

Beispiel 41:

In einer Stickstoffatmosphäre wird ein Gemisch von Magnesiumspänen (36,1 mg, 1,5 mMol) und 5-(4-Chlorbutyl)-imidazo[1,5-a]pyridin (313 mg) in 0,2 ml trockenem Tetrahydrofuran mit einem Jodkristall versetzt. Nach der Auflösung von Magnesiumspänen gibt man weitere 2 ml wasserfreies Tetrahydrofuran und Bromessigsäure-äthylester (0,43 g) dazu. Das Reaktionsgemisch wird bei Raumtemperatur eine Stunde gerührt, 30 Minuten unter Rückfluss gekocht, auf 25° gekühlt, mit 20 ml Essigsäureäthylester verdünnt und mit Wasser (2×10 ml) gewaschen. Die organische Phase wird getrocknet, filtriert und eingedampft. Man erhält das 5-(5-Äthoxycarbonylpentyl)-imidazo[1,5-a]pyridin als ein Öl. Dieses wird in 10 ml Methanol und 5 ml 1-normaler Natriumhydroxidlösung 3 Stunden unter Rückfluss gekocht. Das Methanol wird abgedampft und der Rückstand in 10 ml Wasser gelöst. Diese Lösung wird mit 10 ml Essigsäureäthylester gewaschen und mit konz. Chlorwasserstoffsäure auf den pH-Wert 6 eingestellt. Nach Extraktion mit Methylenchlorid (5×10 ml), Trocknen über Natriumsulfat/Magnesiumsulfat und Eindampfen erhält man das 5-(5-Carboxypentyl)-imidazo-[1,5-a]pyridin des Beispiels 2.

Beispiel 42:

Ein Gemisch von Magnesiumspänen (36,5 mg) und 5-(5-Chlorpentyl)-imidazo[1,5-a]pyridin (313 mg) in 0,2 ml wasserfreiem Tetrahydrofuran wird unter Stickstoff mit einem Jodkristall versetzt. Nach der Auflösung von Magnesiumspänen gibt man weitere 2 ml Tetrahydrofuran dazu. Die Lösung wird auf -5° gekühlt und unter starkem Rühren trockenes Kohlendioxidgas 30 Minuten eingeleitet. Das Lösungsmittel wird abgedampft und der Rückstand in 10 ml 25%iger Schwefelsäure gelöst. Die Lösung wird mit 5 ml Äther gewaschen, auf den pH-Wert 6 angesäuert, mit Methylenchlorid (4×15 ml) extrahiert. Die Extrakte werden über Magnesiumsulfat getrocknet und eingedampft. Man erhält das 5-(5-Carboxypentyl)-imidazo[1,5-a]pyridin des Beispiels 2.

Beispiel 43:

Ein Gemisch von 5-(6-Carboxy-6-oxo-hexyl)-imidazo[1,5-a]pyridin (0,52 g) and 0,5 g Glaspulver wird stufenweise auf 240° erhitzt. Das Reaktionsgemisch wird 1 Stunde bei 240° gehalten und dann auf Raumtemperatur gekühlt. Der Rückstand wird in Methylenchlorid aufgenommen und das feste Material abfiltriert. Eindampfen und Umkristallisation ergibt das 5-(5-Carboxypentyl)-imidazo[1,5-a]pyridin des Beispiels 2.

Der Ausgangsstoff wird wie folgt hergestellt: 5-(5-Chlorpentyl)-imidazo[1,5-a]pyridin wird in Dimethylformamid gelöst, mit 2-Äthoxycarbonyl-1,3-dithian und Natriumhydrid umgesetzt, dann mit N-Bromsuccinimid in wässerigem Aceton behandelt und mit verdünnter Natriumhydroxidlösung hydrolysiert. Man erhält das 5-(6-Carboxy-6-oxohexyl)-imidazo[1,5-a]pyridin.

Beispiel 44:

Eine Lösung von 5-(4-Carboxybutyl)-imidazo[1,5-a]pyridin (0,22 g) und Oxalylchlorid (0,2 g) in 10 ml Chloroform wird 1,5 Stunden unter Rückfluss gekocht. Das Lösungsmittel wird abgedampft, der Rückstand in 15 ml frisch destilliertem trockenem Dioxan gelöst. Unter Kühlung bei 0° und darunter wird die Lösung zu einer äquimolaren ätherischen Lösung von Diazomethan gegeben. Das Gemisch wird über Nacht bei Raumtemperatur stehen gelassen und der Äther vorsichtig abgedampft. Eine Lösung von Silberoxid (0,14 g) in 1 ml 0,84-molarem Natriumthiosulfat wird zu der Lösung der erhaltenen Diazoverbindung gegeben. Das Gemisch wird 3 Stunden bei Zimmertemperatur gerührt und portionenweise mit einer weiteren Silberoxidmenge (0,14 g) versetzt. Das Gemisch wird dann 1 Stunde bei 50° gerührt, abgekühlt, filtriert und mit 1%iger wässeriger Natriumhydroxidlösung versetzt. Ansäuern der wässerigen Phase mit konz. Schwefelsäure, Extraktion mit Methylenchlorid, Trocknen des Extrakts über Magnesiumsulfat und Eindampfen ergibt das 5-(5-Carboxypentyl)-imidazo[1,5-a]pyridin des Beispiels 2.

Beispiel 45:

Ein Gemisch von 4,1 g 5-(5-Hydroxypentyl)-imidazo[1,5-a]pyridin, 1,5 ml Wasser, 1,7 g Nickelcarbonyl, 0,5 g Nickelchloridhexahydrat und 0,3 ml konz. Chlorwasserstoffsäure wird 10 Stunden unter Kohlenmonoxid unter hohem Druck erhitzt. Dann werden alle flüchtigen Stoffe abgedampft. Die zurückgebliebene wässerige Phase wird mit Äther (5 ml) gewaschen, mit 6-normaler Natriumhydroxidlösung auf den pH-Wert 10 eingestellt und wieder mit Äther (10 ml) extrahiert. Ansäuern auf den pH-Wert 6, Extraktion mit Methylenchlorid, Eindampfen und Umkristallisation aus Chloroform/Äther ergibt das 5-(5-Carboxypentyl)-imidazo[1,5-a]pyridin des Beispiels 2.

In analoger Weise ergibt die Behandlung von 5-(4-Pentenyl)-imidazo[1,5-a]pyridin mit Nickelcarbonyl auch das 5-(5-Carboxypentyl)-imidazo[1,5-a]pyridin.

Beispiel 46:

Ein Gemisch von Silbernitrat (0,34 g) in 10 ml Wasser und 5-(5-Formylpentyl)-imidazo[1,5-a]-pyridin (0,2 g) in 10 ml Dioxan wird mit 1-normaler Natriumhydroxidlösung auf den pH-Wert 10 eingestellt und 1 Stunde milde auf 70–80° erwärmt. Das ausgeschiedene Silber wird durch Kieselgur abfiltriert und das Volumen des Filtrats auf 50% eingeengt. Die erhaltene wässerige Base wird mit Essigsäureäthylester extrahiert, mit konz. Schwefelsäure auf den pH-Wert 6 gebracht und mit Methylenchlorid (5×10 ml) extrahiert. Nach Trocknen über Natriumsulfat/Magnesiumsulfat und Eindampfen erhält man das 5-(5-Carboxypentyl)-imidazo[1,5-a]pyridin.

Das als Ausgangsstoff verwendete 5-(5-Formylpentyl)-imidazo[1,5-a]pyridin wird wie folgt hergestellt:

5-(6-Chlorhexyl)-imidazo[1,5-a]pyridin wird mit Dimethylsulfoxid, Triäthylamin und Silbertetrafluoroborat gemäss der in Tetrahedron Letters 1974, 917 beschriebenen Methode behandelt.

Beispiel 47:

Man leitet Ozon in eine Lösung von 5-(6,6-Dimethoxyhexyl)-imidazo[1,5-a]pyridin (0,456 g) in

20 ml Methylenchlorid 4 Stunden bei -50° ein. Das überschüssige Ozon wird mit Stickstoff ausgetrieben. Das Gemisch wird mit 1 ml Dimethylsulfid bei -78° versetzt und man lässt es sich langsam auf Raumtemperatur erwärmen. Das Lösungsmittel wird abgedampft, der Rückstand in 10 ml Methanol aufgenommen und mit 10 ml 1-normaler Natriumhydroxidlösung 2 Stunden unter Rückfluss gekocht. Das Methanol wird abgedampft und der Rückstand mit Essigsäureäthylester (5 ml) gewaschen und mit konz. Schwefelsäure auf den pH-Wert 6 gebracht. Extraktion mit Methylenchlorid (5×10 ml), Trocknen über Magnesiumsulfat und Eindampfen ergibt das 5-(5-Carboxypentyl)-imidazo[1,5-a]pyridin.

Beispiel 48:

2-(N-Formylaminomethyl)-6-(5-äthoxycarbonylpentyl)-pyridin (1,0 g) wird mit 0,25 ml Phosphoroxychlorid in 10 ml Toluol 15 Stunden bei 90° erhitzt. Das überschüssige Phosphoroxychlorid wird mit Toluol abgedampft. Der Rückstand wird mit gesättigter Ammoniumhydroxidlösung bei 0° basisch gemacht und mit Methylenchlorid (4×50 ml) extrahiert. Die Extrakte werden über Natriumsulfat getrocknet und chromatographiert (40 g Silicagel, Essigsäureäthylester). Man erhält das 5-(5-Äthoxycarbonylpentyl)-imidazo[1,5-a]pyridin.

Der Ausgangsstoff wird gemäss den in den vorhergehenden Beispielen beschriebenen Methoden über 6-(5-Äthoxycarbonylpentyl)-2-cyanpyridin hergestellt.

Beispiel 49:

Eine Lösung von Lithiumdiisopropylamid (aus 1,0 g Diisopropylamin und 6,9 ml 1,6-normalem n-Butyllithium) und Hexamethylphosphoramid (1,8 g) in 50 ml Tetrahydrofuran wird auf -50° gekühlt und tropfenweise mit Propiolsäure (0,35 g) versetzt. Man lässt das Reaktionsgemisch sich langsam (in 2 Stunden) auf -15° erwärmen und versetzt es tropfenweise, innerhalb 15 Minuten, mit 5-(4-Chlorbutyl)-imidazo[1,5-a]pyridin (1,04 g) in 10 ml Tetrahydrofuran. Die Kühlung wird entfernt und das Reaktionsgemisch 90 Minuten bei Zimmertemperatur gerührt. Das Gemisch wird in 100 g Eis gegossen, die wässerige Phase abgetrennt, mit Essigester (20 ml) gewaschen, mit konz. Schwefelsäure auf den pH-Wert 2 gebracht und wieder mit Essigsäureäthylester (20 ml) gewaschen. Die wässerige Phase wird auf den pH-Wert 6 eingestellt und mit Methylenchlorid (5×30 ml) extrahiert. Die Extrakte werden über Natriumsulfat/Magnesiumsulfat getrocknet, filtriert und eingedampft. Man erhält das 5-(6-Carboxyhex-5-ynyl)-imidazo[1,5-a]pyridin.

Beispiel 50:

Eine Lösung von 5-Methylimidazo[1,5-a]pyridin (4,0 g, 0,03 Mol) und Tetramethylendiamin (4,9 g) in 100 ml Tetrahydrofuran wird unter Stickstoff auf 0° gekühlt und tropfenweise mit 26,5 ml 1,6-normalem n-Butyllithium versetzt, wobei man die Temperatur unter 5° hält. Nach 40 Minuten wird diese Lösung zu einer eiskalten Lösung von 4-Bromcrotonsäure-äthylester (7,02 g) in 90 ml Tetrahydrofuran gegeben. Nach 15 Minuten wird das Reaktionsgemisch mit überschüssiger gesättigter Ammoniumchloridlösung zerlegt und zwischen Wasser (100 ml) und Essigsäureäthylester (150 ml) verteilt. Die organische Phase wird über Natriumsulfat getrocknet, filtriert und eingedampft. Man erhält das 5-(4-Äthoxycarbonyl-but-3-enyl)-imidazo[1,5-a]pyridin.

Beispiel 51:

Eine Lösung von 5-Methylimidazo[1,5-a]pyridin (4,0 g) und Tetramethylendiamin (4,9 g) in 100 ml Tetrahydrofuran wird unter Stickstoff auf 0° gekühlt und tropfenweise mit 26,5 ml 1,6-normalem n-Butyllithium versetzt, wobei man die Temperatur unter 5° hält. Nach 40 Minuten gibt man diese Lösung zu einer eiskalten Lösung von 1-Tetrahydropyranyloxy-8-brom-oct-6-yn (10,4 g) in 80 ml Tetrahydrofuran. Nach 30 Minuten wird die Reaktion mit 50 ml 2-normaler Chlorwasserstoffsäure unterbrochen und das Gemisch weitere 2 Stunden bei Zimmertemperatur gerührt. Die Schichten werden getrennt und die wässerige Phase mit 50%iger Natriumhydroxidlösung auf den pH-Wert 10 gebracht. Extraktion mit Methylenchlorid (3×30 ml), Trocknen über Natriumsulfat, Filtrieren, Eindampfen und Chromatographie (Silicagel, Essigsäureäthylester) ergibt das 5-(9-Hydroxynon-7-ynyl)-imidazo[1,5-a]pyridin.

Beispiel 52:

Eine Lösung von 150 mg 5-(4-Carboxybuta-1,3-dienyl)-imidazo[1,5-a]pyridin in 9 ml Methanol wird mit 100 mg 10%igem Palladium-auf-Kohle-Katalysator versetzt. Das Reaktionsgemisch wird bei atmosphärischem Druck 2 Stunden hydriert. Der Katalysator wird abfiltriert und das Lösungsmittel unter vermindertem Druck eingedampft. Man erhält das 5-(4-Carboxybutal)-imidazo[1,5-a]pyridin, welches mit der Verbindung des Beispiels 8 identisch ist.

Der Ausgangsstoff wird wie folgt hergestellt:

Eine Lösung von 18 g 3-Äthylthioimidazo[1,5-a]pyridin (Blatcher and Middlemiss, Tet. Lett. (21), 2195 (1980)] in 200 ml Tetrahydrofuran wird bei -50° mit einer Lösung von 80 ml 1,6-molaren n-Butyllithium in Hexan innerhalb 30 Minuten tropfenweise versetzt. Nach der Zugabe wird das Reaktionsgemisch bei -50° weitere 45 Minuten gerührt und die gekühlte Lösung innerhalb 10 Minuten tropfenweise mit 10 ml Dimethylformamid versetzt. Dann lässt man das Reaktionsgemisch sich auf Raumtemperatur erwärmen und giesst es in 500 ml Eiswasser. Das Gemisch wird mit 500 ml Diäthyläther extrahiert. Der Ätherextrakt wird über wasserfreiem Magnesiumsulfat getrocknet, filtriert und unter vermindertem Druck eingedampft. Der ölige Rückstand wird durch Säulenchromatographie auf Silicagel gereinigt und mit einem Gemisch von Diäthyläther-Hexan (1:2) eluiert. Das Lösungsmittel wird abgedampft. Man erhält das 5-Formyl-3-äthylthioimidazo[1,5-a]pyridin. F. 41–43°.

Eine Lösung von 20 g 5-Formyl-3-äthylthioimidazo[1,5-a]pyridin in 200 ml Isopropanol wird mit ungefähr 15 g Raney-Nickel versetzt. Das Reaktionsgemisch wird gerührt und 16 Stunden unter Rückfluss gekocht. Der Katalysator wird durch Kieselgur abfiltriert. Das Filtrat wird unter vermindertem Druck eingedampft. Der erhaltene ölige Rückstand wird durch Säulenchromatographie auf Silicagel gereinigt und mit einem Gemisch von Diäthyläther-Essigsäureäthylester (2:1) eluiert. Das Lösungsmittel wird unter vermindertem Druck abgedampft. Man erhält das 5-Formylimidazo[1,5-a]pyridin, welches bei 138–140° schmilzt.

Eine gerührte Suspension von 150 mg Natriumhydrid in 25 ml Toluol wird mit 550 mg Triäthyl-4-phosphonocrotonat innerhalb 10 Minuten tropfenweise versetzt. Das Reaktionsgemisch wird durch Kühlung mit einem Eisbad auf 5° gehalten. Dann wird das Gemisch mit 300 mg 5-Formylimidazo[1,5-a]pyridin versetzt und bei Raumtemperatur 1 Stunde gerührt. Das Reaktionsgemisch wird in 100 ml Eiswasser gegossen und 2×100 ml Essigsäureäthylester extrahiert. Die Extrakte werden vereinigt, über Magnesiumsulfat getrocknet, filtriert und zur Trockene eingedampft. Der erhaltene ölige Rückstand wird durch Säulenchromatographie auf Silicagel gereinigt und mit einem Gemisch von Diäthyläther-Essigsäureäthylester eluiert. Das Lösungsmittel wird unter vermindertem Druck abgedampft. Man erhält das 5-(4-Äthoxycarbonylbuta-1,3-dienyl)-imidazo[1,5-a]-pyridin, welches bei 101–103° schmilzt.

Eine Lösung von 200 mg 5-(4-Äthoxycarbonylbuta-1,3-dienyl)-imidazo[1,5-a]pyridin in 20 ml Methanol wird mit 4 ml 1-normaler Natriumhydroxidlösung versetzt. Das Reaktionsgemisch wird 18 Stunden bei Raumtemperatur gerührt. Das Methanol wird unter vermindertem Druck abgedampft, der Rückstand mit 20 ml Wasser verdünnt und die Lösung mit Chlorwasserstoffsäure auf den pH-Wert 5 eingestellt. Der Niederschlag wird abgetrennt. Man erhält das 5-(4-Carboxybuta-1,3-dienyl)-imidazo[1,5-a]pyridin, welches bei 243–245° schmilzt.

Effekt auf Thromboxan-Synthetase der menschlichen Blutplättchen

Die Methode wird gemäss der vorne angegebenen Beschreibung durchgeführt. Diese in vitro Hemmung des Thromboxan-Synthetase-Enzyms ist analog zu der Methode von Sun, Biochem. Biophys. Res. Comm. 74, 1432 (1977) gezeigt worden.

Ergebnisse:

Verbindungen der Formel

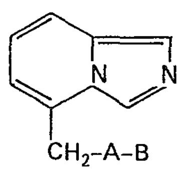

sind in der unten angegebenen Tabelle:

| Verbindung des Beispiels Nr. | $-CH_2-A-B$ | $IC_{50}$ (nM) im zellfreien Testsystem<br>Thromboxan Synthetase |
|---|---|---|
| 8 | $-(CH_2)_4COOH$ | 41 |
| 2 | $-(CH_2)_5COOH$ | 3 |
| 3/b | $-(CH_2)_6COOH$ | 5 |
| 3/c | $-(CH_2)_7COOH$ | 21 |
| 16 | $-(CH_2)_3C(CH_3)_2CH_2COOH$ | 18 |
| 10 | $-(CH_2)_5CONH_2$ | 77 |
| 11 | $-(CH_2)_5CONHCH_3$ | 270 |
| 12 | $-(CH_2)_5CON(CH_3)_2$ | 550 |
| 5 | $-(CH_2)_4-CN$ | 1,500 |
| 4 | $-(CH_2)_5-CN$ | 630 |
| 13 | $-(CH_2)_5CH_2OH$ | 280 |
| 1 | $-(CH_2)_5COOC_2H_5$ | 330 |
| 15/c | 5,6,7,8-Tetrahydro- $-(CH_2)_4COOH$ | 290 |

| Verbindung des Beispiels Nr. | $-CH_2-A-B$ | $IC_{50}$ (nM) im zellfreien Testsystem |
|---|---|---|
| | | Thromboxan Synthetase |
| 15/b | 5,6,7,8-Tetrahydro- $-(CH_2)_5COOH$ | 85 |
| 15/a | 5,6,7,8-Tetrahydro- $-(CH_2)_6COOH$ | 14 |

**Patentansprüche für die Vertragsstaaten BE, CH, DE, FR, IT, LI, LU, NL, SE**

1. Verbindungen der allgemeinen Formel (I)

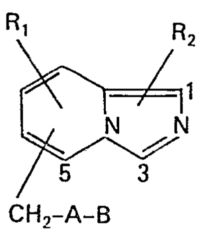

I

oder ihre 5,6,7,8-Tetrahydroderivate, worin jedes der Symbole $R_1$ und $R_2$ Wasserstoff, Halogen oder $C_1$-$C_7$-Alkyl bedeutet, A für ein Alkylen mit 1 bis 12 Kohlenstoffatomen, Alkynylen oder Alkenylen mit 2 bis 12 Kohlenstoffatomen steht, B Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl bedeutet, und ihre Salze.

2. Verbindungen der im Anspruch 1 gezeigten Formel (I) in welchen die Gruppe $CH_2$-A-B an die 5-Stellung gebunden ist, und ihre Salze.

3. Verbindungen der Formel (II)

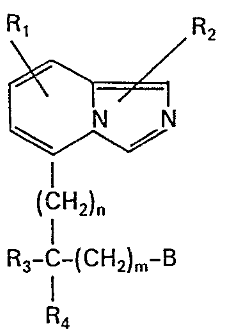

II

oder ihre 5,6,7,8-Tetrahydroderivate, worin jedes der Symbole $R_1$, $R_2$, $R_3$ und $R_4$ Wasserstoff oder $C_1$-$C_7$-Alkyl bedeutet, n eine ganze Zahl von 1 bis 7 ist, m Null oder 1 ist, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze.

4. Verbindungen der Formel (III)

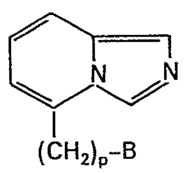

III

oder ihre 5,6,7,8-Tetrahydroderivate, worin p eine ganze Zahl von 3 bis 8 bedeutet, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze.

5. Verbindungen der Formel (IV)

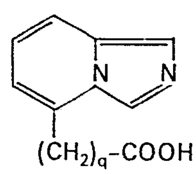

IV

oder ihre 5,6,7,8-Tetrahydroderivate, worin q 4, 5 oder 6 bedeutet, und ihre Salze.

6. 5-(5-Carboxypentyl)-imidazo[1,5-a]pyridin und seine Salze.

7. 5-(6-Carboxyhexyl)-imidazo[1,5-a]pyridin und seine Salze.

8. 5-(6-Carboxyhexyl)-5,6,7,8-tetrahydroimidazo[1,5-a]pyridin und seine Salze.

9. 5-(7-Carboxyheptyl)-imidazo[1,5-a]pyridin und seine Salze.

10. 5-(5-Carboxy-4,4-dimethylpentyl)-imidazo-[1,5-a]pyridin und seine Salze.

11. Pharmazeutische Präparate enthaltend Verbindungen der Ansprüche 1 bis 10 oder ihre Salze zusammen mit einem pharmazeutischen Trägermaterial.

12. Die in den Ansprüchen 1 bis 10 genannten Verbindungen zur Anwendung in einem Verfahren zur therapeutischen Behandlung des menschlichen oder tierischen Körpers.

13. Die in den Ansprüchen 1 bis 10 genannten Verbindungen zur Anwendung als Hemmer der Thromboxan-Synthetase.

14. Verwendung der in den Ansprüchen 1 bis 10 genannten Verbindungen zur Herstellung von pharmazeutischen Präparaten.

15. Verfahren zur Herstellung von den im Anspruch 1 genannten Verbindungen der Formel (I) und ihren Salzen, dadurch gekennzeichnet, dass man entweder

a) eine Verbindung der Formel (VI)

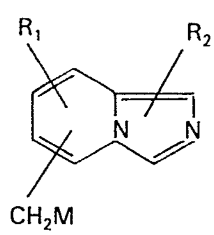

VI

worin M ein Alkalimetall bedeutet, jedes der Symbole $R_1$ und $R_2$ für Wasserstoff oder $C_1$-$C_7$-Alkyl steht, mit einem reaktionsfähigen funktionellen Derivat einer Verbindung der Formel (VII)

HO–A–B′ VII

worin A die oben angegebene Bedeutung hat, B′ Carboxy, Trialkoxymethyl, unsubstituiertes, Mono- oder Di-($C_1$-$C_7$-alkyl)-substituiertes Carbamoyl, Cyan, veräthertes Hydroxymethyl oder Halogenmethyl bedeutet, umsetzt, und, eine erhaltene Verbindung der Formel (Ia)

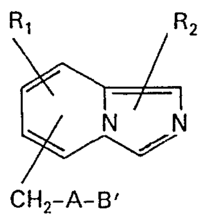

Ia

worin sich B′ von B unterscheidet, in die Verbindung der Formel (I) umwandelt, oder

b) eine Verbindung der Formel (VIII)

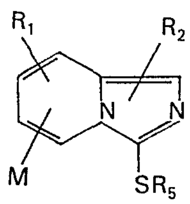

VIII

worin M ein Alkalimetall bedeutet, jedes der Symbole $R_1$ und $R_2$ für Wasserstoff oder $C_1$-$C_7$-Alkyl steht, und $R_5$ $C_1$-$C_7$-Alkyl bedeutet, mit einem reaktionsfähigen funktionellen Derivat einer Verbindung der Formel (IX)

$HOCH_2$–A–B′ IX

worin A die oben angegebene Bedeutung hat, B′ Carboxy, Trialkoxymethyl, unsubstituiertes, Mono- oder Di-($C_1$-$C_7$-alkyl)-substituiertes Carbamoyl, Cyan, veräthertes Hydroxymethyl oder Halogenmethyl bedeutet, umsetzt, in einer erhaltenen Verbindung, worin sich B′ von B unterscheidet, die Gruppe B′ in B umwandelt, und die erhaltene Verbindung desulfuriert, oder

c) unter basischer Katalyse eine Verbindung der allgemeinen Formel (X)

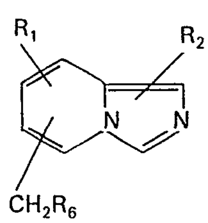

X

worin jedes der Symbole $R_1$ und $R_2$ Wasserstoff oder $C_1$-$C_7$-Alkyl bedeutet, $R_6$ für $C_1$-$C_7$-Alkoxycarbonyl oder Cyan steht, mit einem reaktionsfähigen funktionellen Derivat einer Verbindung der Formel (VII)

HO–A–B′ VII

worin A die vorher angegebene Bedeutung hat, B′ für Carboxy, Trialkoxymethyl, unsubstituiertes, Mono- oder Di-($C_1$-$C_7$-alkyl) substituiertes Carbamoyl, Cyan, veräthertes Hydroxymethyl oder Halogenmethyl steht, umsetzt, die Gruppe $R_6$ abspaltet und eine erhaltene Verbindung der Formel (I) umwandelt, oder

d) eine Verbindung der Formel (XI)

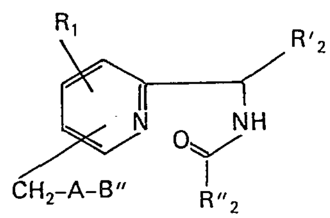

XI

worin jedes der Symbole $R_1$, $R_2'$ und $R_2''$ Wasserstoff oder $C_1$-$C_7$-Alkyl bedeutet, A die oben angegebene Bedeutung hat, und B″ für Carboxy, $C_1$-$C_7$-Alkoxycarbonyl, unsubstituiertes, Mono- oder Di-($C_1$-$C_7$-alkyl)-substituiertes Carbamoyl, Cyan, Hydroxymethyl, $C_1$-$C_7$-Alkanoyloxymethyl, veräthertes Hydroxymethyl oder Halomethyl steht, zu einer Verbindung der Formel (Ib)

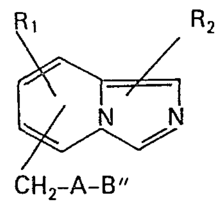

Ib

ringschliesst, und eine erhaltene Verbindung, worin sich B″ von B unterscheidet, in eine Verbindung der Formel (I) umwandelt, oder

e) eine Verbindung der Formel (XIV)

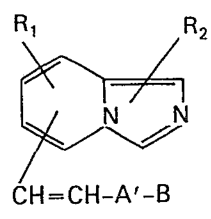

XIV

worin A′ einen Alkylen-, Alkenylen- oder Alkynylenrest mit höchstens 11 Kohlenstoffatomen bedeutet, hydriert, oder

f) in einer Verbindung der Formel (XVI)

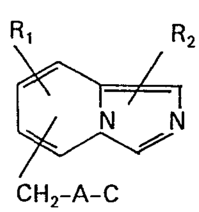

XVI

worin $R_1$, $R_2$ und A die vorher angegebene Bedeutung haben, und C einen in eine Carboxygruppe überführbaren Rest bedeutet, die Gruppe C, gegebenenfalls unter Verlängerung der Kette A innerhalb ihrer Definition, in die Carboxygruppe überführt,

und, wenn erwünscht, eine erhaltene Verbindung der Formel (I) in eine andere Verbindung der Erfindung umwandelt,

und/oder, wenn erwünscht, eine erhaltene freie Verbindung in ein Salz oder ein erhaltenes Salz in die freie Verbindung oder in ein anderes Salz überführt,

und/oder, wenn erwünscht, ein erhaltenes Gemisch von Isomeren oder Razematen in die einzelnen Isomeren oder Razemate auftrennt,

und/oder, wenn erwünscht, erhaltene Razemate in die optischen Antipoden aufspaltet.

16. Die nach dem Verfahren des Anspruchs 15 erhältlichen Verbindungen.

**Patentansprüche für den Vertragsstaat: AT**

1. Verfahren zur Herstellung von neuen Imidazo[1,5-a]pyridinen der allgemeinen Formel (I)

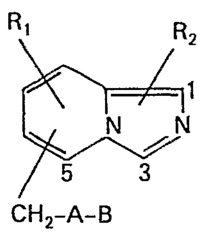

I

oder ihren 5,6,7,8-Tetrahydroderivaten, worin jedes der Symbole $R_1$ und $R_2$ Wasserstoff, Halogen oder $C_1$-$C_7$-Alkyl bedeutet, A für ein Alkylen mit 1 bis 12 Kohlenstoffatomen, Alkynylen oder Alkenylen mit 2 bis 12 Kohlenstoffatomen steht, B Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl bedeutet und ihren Salzen, dadurch gekennzeichnet, dass man entweder

a) eine Verbindung der Formel (VI)

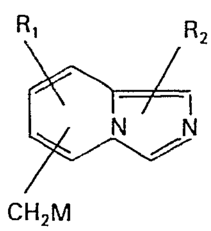

VI

worin M ein Alkalimetall bedeutet, jedes der Symbole $R_1$ und $R_2$ für Wasserstoff oder $C_1$-$C_7$-Alkyl steht, mit einem reaktionsfähigen funktionellen Derivat einer Verbindung der Formel (VII)

HO-A-B' VII

worin A die oben angegebene Bedeutung hat, B' Carboxy, Trialkoxymethyl, unsubstituiertes, Mono- oder Di-($C_1$-$C_7$-alkyl)-substituiertes Carbamoyl, Cyan, veräthertes Hydroxymethyl oder Halogenmethyl bedeutet, umsetzt, und, eine erhaltene Verbindung der Formel (Ia)

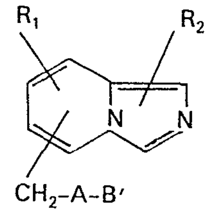

Ia

worin sich B' von B unterscheidet, in die Verbindung der Formel (I) umwandelt,

b) eine Verbindung der Formel (VIII)

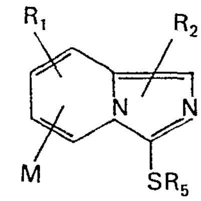

VIII

worin M ein Alkalimetall bedeutet, jedes der Symbole $R_1$ und $R_2$ für Wasserstoff oder $C_1$-$C_7$-Alkyl steht, und $R_5$ $C_1$-$C_7$-Alkyl bedeutet, mit einem reaktionsfähigen funktionellen Derivat einer Verbindung der Formel (IX)

$HOCH_2$-A-B' IX

worin A die oben angegebene Bedeutung hat, B' Carboxy, Trialkoxymethyl, unsubstituiertes, Mono- oder Di-($C_1$-$C_7$-alkyl)-substituiertes Carbamoyl, Cyan, veräthertes Hydroxymethyl oder Halogenmethyl bedeutet, umsetzt, in einer erhaltenen Verbindung, worin sich B' von B unterscheidet, die Gruppe B' in B umwandelt, und die erhaltene Verbindung desulfuriert,

c) unter basischer Katalyse eine Verbindung der allgemeinen Formel (X)

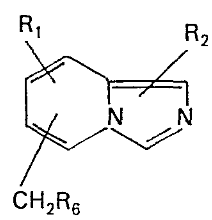

X

worin jedes der Symbole $R_1$ und $R_2$ Wasserstoff oder $C_1$-$C_7$-Alkyl bedeutet, $R_6$ für $C_1$-$C_7$-Alkoxycarbonyl oder Cyan steht, mit einem reaktionsfähigen funktionellen Derivat einer Verbindung der Formel (VII)

HO-A-B' VII

worin A die vorher angegebene Bedeutung hat, B' für Carboxy, Trialkoxymethyl, unsubstituiertes, Mono- oder Di-($C_1$-$C_7$-alkyl)-substituiertes Carbamoyl, Cyan, veräthertes Hydroxymethyl oder Halogenmethyl steht, umsetzt, die Gruppe $R_6$ abspaltet und eine erhaltene Verbindung, worin sich B′ von B unterscheidet, in eine Verbindung der Formel (I) umwandelt,

d) eine Verbindung der Formel (XI)

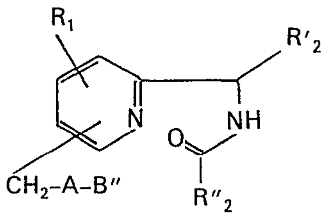

XI

worin jedes der Symbole $R_1$, $R'_2$ und $R''_2$ Wasserstoff oder $C_1$-$C_7$-Alkyl bedeutet, A die oben angegebene Bedeutung hat, und B″ für Carboxy, $C_1$-$C_7$-Alkoxycarbonyl, unsubstituiertes, Mono- oder Di-($C_1$-$C_7$-alkyl)-substituiertes Carbamoyl, Cyan, Hydroxymethyl, $C_1$-$C_7$-Alkanoyloxymethyl, veräthertes Hydroxymethyl oder Halomethyl steht, zu einer Verbindung der Formel (Ib)

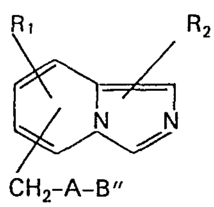

Ib

ringschliesst, und eine erhaltene Verbindung, worin sich B″ von B unterscheidet, in eine Verbindung der Formel (I) umwandelt,

e) eine Verbindung der Formel (XIV)

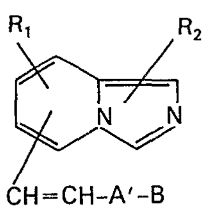

XIV

worin A′ einen Alkylen-, Alkenylen- oder Alkynylenrest mit höchstens 11 Kohlenstoffatomen bedeutet, hydriert, oder

f) in einer Verbindung der Formel (XVI)

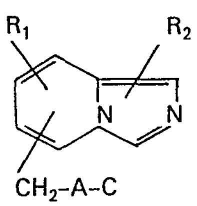

XVI

worin $R_1$, $R_2$ und A die vorher angegebene Bedeutung haben, und C einen in eine Carboxygruppe überführbaren Rest bedeutet, die Gruppe C, gegebenenfalls unter Verlängerung der Kette A innerhalb ihrer Definition, in die Carboxygruppe überführt,

und, wenn erwünscht, eine erhaltene Verbindung der Formel I in eine andere Verbindung der Erfindung umwandelt,

und/oder, wenn erwünscht, eine erhaltene freie Verbindung in ein Salz oder ein erhaltenes Salz in die freie Verbindung oder in ein anderes Salz überführt,

und/oder, wenn erwünscht, ein erhaltenes Gemisch von Isomeren oder Razematen in die einzelnen Isomeren oder Razemate auftrennt,

und/oder, wenn erwünscht, erhaltene Razemate in die optischen Antipoden aufspaltet.

2. Verfahren nach Anspruch 1, dadurch gekennzeichnet, dass man Verbindungen der im Anspruch 1 gezeigten Formel (I), in welchen die Gruppe $CH_2$-A-B an die 5-Stellung gebunden ist, und ihre Salze herstellt.

3. Verfahren nach Anspruch 1, dadurch gekennzeichnet, dass man Verbindungen der allgemeinen Formel (II)

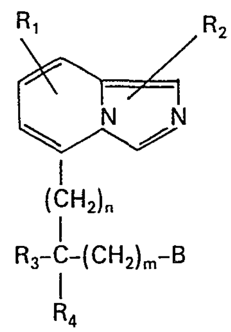

II

oder ihre 5,6,7,8-Tetrahydroderivate, worin jedes der Symbole $R_1$, $R_2$, $R_3$ und $R_4$ Wasserstoff oder $C_1$-$C_4$-Alkyl bedeutet, n eine ganze Zahl von 1 bis 7 ist, m Null oder 1 ist, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze herstellt.

4. Verfahren nach Anspruch 1, dadurch gekennzeichnet, dass man Verbindungen der Formel (III)

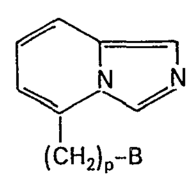

III

oder ihre 5,6,7,8-Tetrahydroderivate, worin p eine ganze Zahl von 3 bis 8 bedeutet, B für Carboxy, $C_1$-$C_7$-Alkoxycarbonyl, unsubstituiertes, Mono- oder Di-($C_1$-$C_7$-alkyl)-substituiertes Carbamoyl, Cyan oder Hydroxymethyl steht, und ihre Salze herstellt.

5. Verfahren nach Anspruch 1, dadurch gekennzeichnet, dass man Verbindungen der Formel (IV)

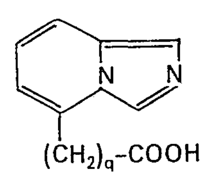

IV

oder ihre 5,6,7,8-Tetrahydroderivate, worin q 4, 5 oder 6 bedeutet, und ihre Salze herstellt.

6. Verfahren nach Anspruch 1, dadurch gekennzeichnet, dass man 5-(5-Carboxypentyl)-imidazo[1,5-a]pyridin und seine Salze herstellt.

7. Verfahren nach Anspruch 1, dadurch gekennzeichnet, dass man 5-(6-Carboxyhexyl)-imidazo[1,5-a]pyridin und seine Salze herstellt.

8. Verfahren nach Anspruch 1, dadurch gekennzeichnet, dass man 5-(6-Carboxyhexyl)-5,6,7,8-tetrahydroimidazo[1,5-a]pyridin und seine Salze herstellt.

9. Verfahren nach Anspruch 1, dadurch gekennzeichnet, dass man 5-(7-Carboxyheptyl)-imidazo[1,5-a]pyridin und seine Salze herstellt.

10. Verfahren nach Anspruch 1, dadurch gekennzeichnet, dass man 5-(5-Carboxy-4,4-dimethylpentyl)-imidazo[1,5-a]pyridin und seine Salze herstellt.

11. Verfahren nach Anspruch 1, dadurch gekennzeichnet, dass man gemäss den Verfahrensvarianten a)-c), jedoch ohne Verbindungen der Formeln (VII) und (IX) zu verwenden, worin B' Cyan bedeutet, die in den Ansprüchen 1 bis 10 genannten Verbindungen und ihre Salze herstellt,

und, wenn erwünscht, eine erhaltene Verbindung der Erfindung in eine andere Verbindung der Erfindung umwandelt,

und/oder, wenn erwünscht, eine erhaltene freie Verbindung in ein Salz oder ein erhaltenes Salz in die freie Verbindung oder in ein anderes Salz überführt,

und/oder, wenn erwünscht, ein erhaltenes Gemisch von Isomeren oder Razematen in die einzelnen Isomeren oder Razemate auftrennt,

und/oder, wenn erwünscht, erhaltene Razemate in die optischen Antipoden aufspaltet.

12. Verfahren zur Herstellung eines pharmazeutischen Präparates, gekennzeichnet durch die Verarbeitung eines erfindungsgemässen Wirkstoffes nach einem der Ansprüche 1 bis 10, mit einem pharmazeutischen Trägermaterial.

13. Verfahren zur Herstellung eines pharmazeutischen Präparates, gekennzeichnet durch die Verarbeitung eines erfindungsgemässen Wirkstoffes nach Anspruch 11 mit einem pharmazeutischen Trägermaterial.

**Claims for the Contracting States: DE, FR, CH, LI, IT, NL, BE, SE, LU**

1. A compound of the general formula I

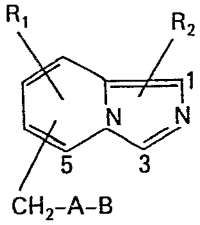

I

or a 5,6,7,8-tetrahydro derivative there of, in which formula each of $R_1$ and $R_2$ is hydrogen, halogen or $C_1$-$C_7$-alkyl, A is $C_1$-$C_{12}$-alkylene, alkynylene or $C_1$-$C_{12}$-alkenylene, B is carboxy, $C_1$-$C_7$-alkoxycarbonyl, carbamoyl which is unsubstituted or substituted by one or two $C_1$-$C_7$-alkyl groups, or is cyano or hydroxymethyl, or a salt thereof.

2. A compound of formula I according to claim 1, wherein the $CH_2$-A-B group is attached in the 5-position, or a salt thereof.

3. A compound of formula II

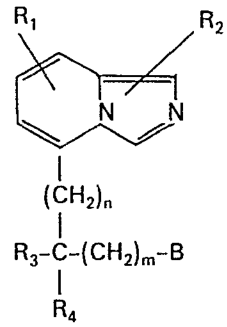

II

or a 5,6,7,8-tetrahydro derivative thereof, in which formula each of $R_1$, $R_2$, $R_3$ and $R_4$ is hydrogen or $C_1$-$C_4$-alkyl, n is an integer from 1 to 7, m is 0 or 1, B is carboxy, $C_1$-$C_7$-alkoxycarbonyl, carbamoyl which is unsubstituted or substituted by one or two $C_1$-$C_7$-alkyl groups, or is cyano or hydroxymethyl, or a salt thereof.

4. A compound of formula III

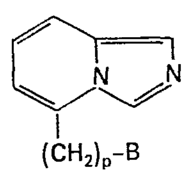

III

or a 5,6,7,8-tetrahydro derivative thereof, in which formula p is an integer from 3 to 8, B is carboxy, $C_1$-$C_7$-alkoxycarbonyl, carbamoyl which is unsubstituted or substituted by one or two $C_1$1-$C_7$-alkyl groups, or is cyano or hydroxymethyl, or a salt thereof.

5. A compound of formula IV

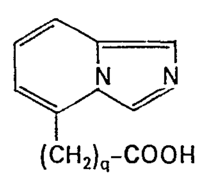

IV

or a 5,6,7,8-tetrahydro derivative thereof, in which formula q is 4, 5 or 6, or a salt thereof.

6. 5-(5-Carboxypentyl)imidazo[1,5-a]pyridine or a salt thereof.

7. 5-(6-Carboxyhexyl)imidazo[1,5-a]pyridine or a salt thereof.

8. 5-(6-Carboxyhexyl)-5,6,7,8-tetrahydroimidazo[1,5-a]pyridine or a salt thereof.

9. 5-(7-Carboxyheptyl)imidazo[1,5-a]pyridine or a salt thereof.

10. 5-(5-Carboxy-4,4-dimethylpentyl)imidazo-[1,5-a]pyridine or a salt thereof.

11. A pharmaceutical composition containing a compound according to any one of claims 1 to 10, or a salt thereof, together with a pharmaceutical carrier.

12. A compound according to any one of claims 1 to 10 for use in a therapeutic method of treating the human or animal body.

13. A compound according to any one of claims 1 to 10 for use as an inhibitor of thromboxane synthetase.

14. Use of a compound according to any one of claims 1 to 10 for the preparation of a pharmaceutical composition.

15. A process for the preparation of a compound of formula I according to claim 1, or of a salt thereof, which process comprises either

a) reacting a compound of formula VI

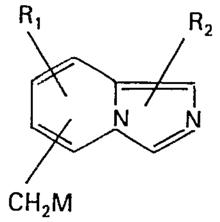

VI

wherein M is an alkali metal, each of $R_1$ and $R_2$ is hydrogen or $C_11$-$C_7$-alkyl, with a reactive functional derivative of a compound of formula VII

HO–A–B′ (VII)

wherein A is as defined above, B′ is carboxy, trialkoxymethyl, carbamoyl which is unsubstituted or substituted by one or two $C_11$-$C_7$-alkyl groups, or is cyano, etherified hydroxymethyl or halomethyl, and converting a resultant compound of formula Ia

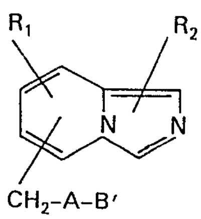

Ia

wherein B′ is different from B, into the compound of formula I, or

b) reacting a compound of formula VIII

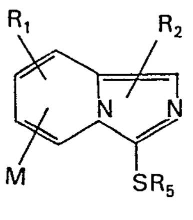

VIII

wherein M is an alkali metal, each of $R_1$ and $R_2$ is hydrogen or $C_11$-$C_7$-alkyl, and $R_5$ is $C_11$-$C_7$-alkyl, with a reactive functional derivative of a compound of formula IX

$HOCH_2$–A–B′ (IX)

wherein A is as defined above, B′ is carboxy, trialkoxymethyl, carbamoyl which is unsubstituted or substituted by one or two $C_11$-$C_7$-alkyl groups, or is cyano, etherified hydroxymethyl or halomethyl, and, in a resultant compound, wherein B′ is different from B, converting the group B′ into B, and desulfurating the resultant compound, or

c) reacting by means of basic catalysis a compound of the general formula X

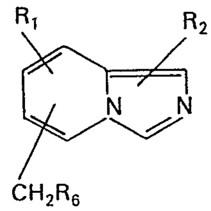

X

wherein each of $R_1$ and $R_2$ is hydrogen or $C_1$-$C_7$-alkyl, $R_6$ is $C_11$-$C_7$-alkoxycarbonyl or cyano, with a reactive functional derivative of a compound of formula VII

HO–A–B′ (VII)

wherein A is as defined above, B′ is carboxy, trialkoxymethyl, carbamoyl which is unsubstituted or substituted by one or two $C_1$-$C_7$-alkyl groups, or is cyano, etherified hydroxymethyl or halomethyl, removing the $R_6$ group and converting a resultant compound, wherein B′ is different from B, into a compound of formula I, or

d) ring-closing a compound of formula XI

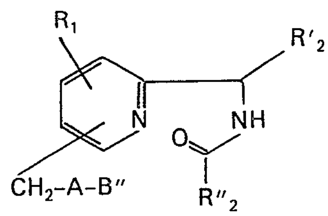

XI

wherein each of $R_1$, $R_2'$ and $R_2''$ is hydrogen or $C_1$-$C_7$-alkyl, A is as defined above, and B″ is carboxy, $C_11$-$C_7$-alkoxycarbonyl, carbamoyl which is unsubstituted or substituted by one or two $C_11$-$C_7$-alkyl groups, or is cyano, hydroxymethyl, $C_11$-$C_7$-alkanoyloxymethyl, etherified hydroxymethyl or halomethyl, to a compound of formula Ib

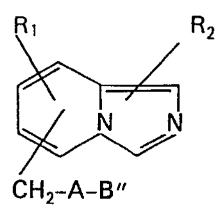

Ib

and converting a resultant compound, wherein B″ is different from B, into a compound of formula I, or

e) hydrogenating a compound of formula XIV

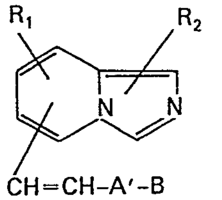

XIV

wherein A′ is an alkylene, alkenylene or alkynylene radical containing not more than 11 carbon atoms, or

f) in a compound of formula XVI

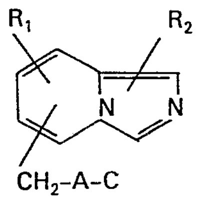

XVI

wherein $R_1$, $R_2$ and A are as defined above, and C is a radical which is convertible into a carboxyl group, converting the group C into the carboxyl group, with or without lengthening of the chain A within the scope of its definition,

and, if desired, converting a resultant compound of formula I into another compound of the invention,

and/or if desired, converting a resultant free compound into a salt or converting a resultant salt into the free compound or into another salt,

and/or, if desired, separating a resultant mixture of isomers or racemates into the individual isomers or racemates,

and/or, if desired, splitting resultant racemates into the optical antipodes.

16. A compound obtainable by the process according to claim 15.

**Claims for the Contracting State: AT**

1. A process for the preparation of a novel imidazo[1,5-a]pyridine of the general formula I

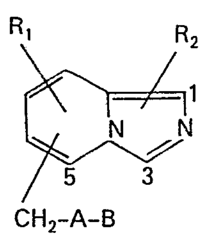

I

or of a 5,6,7,8-tetrahydro derivative thereof, in which formula each of $R_1$ and $R_2$ is hydrogen, halogen or $C_1$-$C_7$-alkyl, A is $C_1$-$C_{12}$-alkylene, alkynylene or $C_1$-$C_{12}$-alkenylene, B is carboxy, $C_1$-$C_7$-alkoxycarbonyl, carbamoyl which is unsubstituted or substituted by one or two $C_1$-$C_7$-alkyl groups, or is cyano or hydroxymethyl, or of a salt thereof, which process comprises either

a) reacting a compound of formula VI

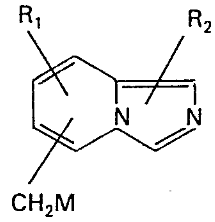

VI

wherein M is an alkali metal, each of $R_1$ and $R_2$ is hydrogen or $C_1$-$C_7$-alkyl, with a reactive functional derivative of a compound of formula VII

HO-A-B′ (VII)

wherein A is as defined above, B′ is carboxy, trialkoxymethyl, carbamoyl which is unsubstituted or substituted by one or two $C_1$-$C_7$-alkyl groups, or is cyano, etherified hydroxymethyl or halomethyl, and converting a resultant compound of formula Ia

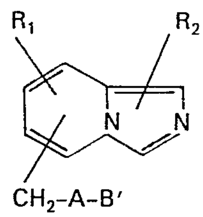

Ia

wherein B′ is different from B, into the compound of formula I,

b) reacting a compound of formula VIII

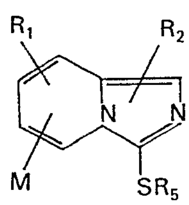

VIII

wherein M is an alkali metal, each of $R_1$ und $R_2$ is hydrogen or $C_1$-$C_7$-alkyl, and $R_5$ is $C_1$-$C_7$-alkyl, with a reactive functional derivative of a compound of formula IX

$HOCH_2$-A-B′ (IX)

wherein A is as defined above, B′ is carboxy, trialkoxymethyl, carbamoyl which is unsubstituted or substituted by one or two $C_1$-$C_7$-alkyl groups, or is cyano, etherified hydroxymethyl or halomethyl, and, in a resultant compound, wherein B′ is different from B, converting the group B′ into B, and desulfurating the resultant compound,

c) reacting by means of basic catalysis a compound of the general formula X

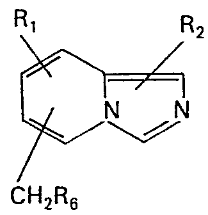

X

wherein each of $R_1$ and $R_2$ is hydrogen or $C_1$-$C_7$-alkyl, $R_6$ is $C_1$-$C_7$-alkoxycarbonyl or cyano, with a reactive functional derivative of a compound of formula VII

HO-A-B′ (VII)

wherein A is as defined above, B′ is carboxy, trialkoxymethyl, carbamoyl which is unsubstituted or substituted by one or two $C_1$-$C_7$-alkyl groups, or is cyano, etherified hydroxymethyl or halomethyl, removing the $R_6$ group and converting a resultant compound, wherein B′ is different from B, into a compound of formula I,

d) ring-closing a compound of formula XI

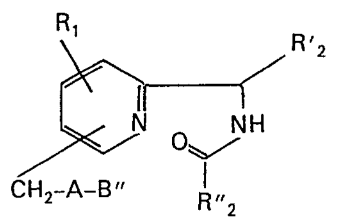

XI

wherein each of $R_1$, $R_2'$ and $R_2''$ is hydrogen or $C_1$-$C_7$-alkyl, A is as defined above, and B″ is carboxy, $C_1$-$C_7$-alkoxycarbonyl, carbamoyl which is unsubstituted or substituted by one or two $C_1$-$C_7$-alkyl groups, or is cyano, hydroxymethyl, $C_1$-$C_7$-alkanoyloxymethyl, etherified hydroxymethyl or halomethyl, to a compound of formula Ib

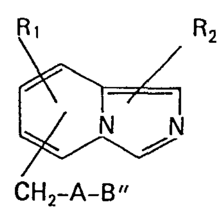

Ib

and converting a resultant compound, wherein B″ is different from B, into a compound of formula I,

e) hydrogenating a compound of formula XIV

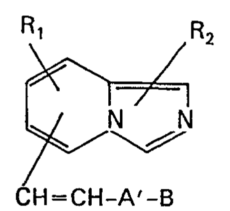

XIV

wherein A′ is an alkylene, alkenylene or alkynylene radical containing not more than 11 carbon atoms, or

f) in a compound of formula XVI

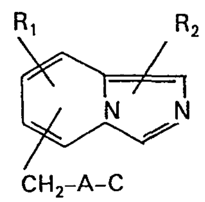

XVI

wherein $R_1$, $R_2$ and A are as defined above, and C is a radical which is convertible into a carboxyl group, converting the group C into the carboxyl group, with or without lengthening of the chain A within the scope of its definition,

and, if desired, converting a resultant compound of formula I into another compound of the invention,

and/or, if desired, converting a resultant free compound into a salt or converting a resultant salt into the free compound or into another salt,

and/or, if desired, separating a resultant mixture of isomers or racemates into the individual isomers or racemates,

and/or, if desired, splitting resultant racemates into the optical antipodes.

2. A process according to claim 1, which process comprises preparing a compound of formula I according to claim 1, wherein the $CH_2$-A-B group is attached in the 5-position, or a salt thereof.

3. A process according to claim 1, which process comprises preparing a compound of the general formula II

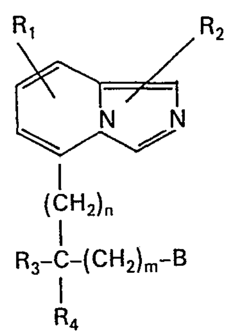

II

or a 5,6,7,8-tetrahydro derivative thereof, in which formula each of $R_1$, $R_2$, $R_3$ and $R_4$ is hydrogen or $C_1$-$C_4$-alkyl, n is an integer from 1 to 7, m is 0 or 1, B is carboxy, $C_1$-$C_7$-alkoxycarbonyl, carbamoyl which is unsubstituted or substituted by one or two $C_1$-$C_7$-alkyl groups, or is cyano or hydroxymethyl, or a salt thereof.

4. A process according to claim 1, which process comprises preparing a compound of formula III

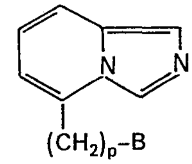

III

or a 5,6,7,8-tetrahydro derivative thereof, in which formula p is an integer from 3 to 8, B is carboxy, $C_1$-$C_7$-alkoxycarbonyl, carbamoyl which is unsubstituted or substituted by one or two $C_1$-$C_7$-alkyl groups, or is cyano or hydroxymethyl, or a salt thereof.

5. A process according to claim 1, which process comprises preparing a compound of formula IV

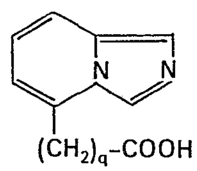

IV

or a 5,6,7,8-tetrahydro derivative thereof, in which formula q is 4, 5 or 6, or a salt thereof.

6. A process according to claim 1, which process comprises preparing 5-(5-carboxypentyl)-imidazo[1,5-a]pyridine or a salt thereof.

7. A process according to claim 1, which process comprises preparing 5-(6-carboxyhexyl) imidazo[1,5-a]pyridine or a salt thereof.

8. A process according to claim 1, which process comprises preparing 5-(6-carboxyhexyl)-5,6,7,8-tetrahydroimidazo[1,5-a]pyridine or a salt thereof.

9. A process according to claim 1, which process comprises preparing 5-(7-carboxyheptyl) imidazo[1,5-a]pyridine or a salt thereof.

10. A process according to claim 1, which process comprises preparing 5-(5-carboxy-4,4-dimethylpentyl)imidazo[1,5-a]pyridine or a salt thereof.

11. A process according to claim 1, which process comprises preparing, in accordance with process variants a) to c) but without using compounds of formulae VII and IX wherein B′ is cyano, a compound according to any one of claims 1 to 10 or a salt thereof,

and, if desired, converting a resultant compound of the invention into another compound of the invention,

and/or, if desired, converting a resultant free compound or into a salt or converting a resultant salt into the free compound or into another salt,

and/or, if desired, separating a resultant mixture of isomers or racemates into the individual isomers or racemates,

and/or, if desired, splitting resultant racemates into the optical antipodes.

12. A process for the preparation of a pharmaceutical composition, which process comprises combining a compound of the invention according to any one of claims 1 to 10 with a pharmaceutical carrier.

13. A process for the preparation of a pharmaceutical composition, which process comprises combining a compound of the invention according to claim 11 with a pharmaceutical carrier.

**Revendications pour les Etats contractants: DE, FR, CH, LI, IT, NL, BE, SE, LU**

1. Composés de formule générale I

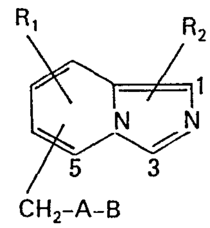

I

ou leurs dérivés 5,6,7,8-tétrahydrogénés, dans lesquels chacun des symboles $R_1$ et $R_2$ représente l'hydrogène, un halogène ou un groupe alkyle en $C_1$-$C_7$, A représente un groupe alkylène en $C_1$-$C_{12}$, alcynylène ou alcénylène en $C_2$-$C_{12}$, B représente un groupe carboxy, (alcoxy en $C_1$-$C_7$)-carbonyle, carbamoyle non substitué, mono- ou disubstitué par des groupes alkyle en $C_1$-$C_7$, cyano ou hydroxyméthyle, et leurs sels.

2. Composés de formule I de la rev. 1, dans lesquels le groupe $CH_2$–A–B est fixé en position 5, et leurs sels.

3. Composés de formule II

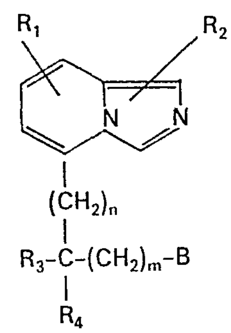

II

ou leurs dérivés 5,6,7,8-tétrahydrogénés, dans lesquels chacun des symboles $R_1$, $R_2$, $R_3$ et $R_4$ représente l'hydrogène ou des groupes alkyle en $C_1$-$C_4$, n est un nombre entier de 1 à 7, m est égal à 0 ou 1, B représente un groupe carboxy, (alcoxy en $C_1$-$C_7$)-carbonyle, carbamoyle non substitué, mono- ou di-substitué par des groupes alkyle en $C_1$-$C_7$, cyano ou hydroxyméthyle, et leurs sels.

4. Composés de formule III

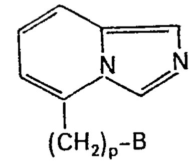

III

ou leurs dérivés 5,6,7,8-tétrahydrogénés, dans lesquels p est un nombre entier de 3 à 8, B représente un groupe carboxy, (alcoxy en $C_1$-$C_7$)-carbonyle, carbamoyle non substitué, mono- ou di-substitué par des groupes alkyle en $C_1$-$C_7$, cyano ou hydroxyméthyle et leurs sels.

5. Composés de formule IV

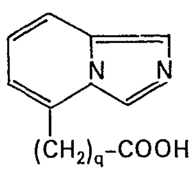

IV

ou leurs dérivés 5,6,7,8-tétrahydrogénés, dans lesquels q est égal à 4, 5 ou 6, et leurs sels.

6. La 5-(5-carboxypentyl)-imidazo[1,5-a]pyridine et ses sels.

7. La 5-(6-carboxyhexyl)-imidazo[1,5-a]pyridine et ses sels.

8. La 5-(6-carboxyhexyl)-5,6,7,8-tétrahydro-imidazo[1,5-a]pyridine et ses sels.

9. La 5-(7-carboxyheptyl)-imidazo[1,5-a]pyridine et ses sels.

10. La 5-(5-carboxy-4,4-diméthylpentyl)-imidazo[1,5-a]pyridine et ses sels.

11. Compositions pharmaceutiques contenant des composés des rev. 1 à 10 ou leurs sels avec un véhicule pharmaceutique.

12. Les composés des rev. 1 à 10, pour l'utilisation dans un procédé pour le traitement thérapeutique de l'organisme humain ou animal.

13. Les composés des rev. 1 à 10, pour l'utilisation en tant qu'inhibiteurs de la thromboxanne-synthétase.

14. Utilisation des composés des rev. 1 à 10, pour la préparation de compositions pharmaceutiques.

15. Procédé de préparation des composés de formule I de la rev. 1 et de leurs sels, caractérisé en ce que:

a) ou bien on fait réagir un composé de formule VI

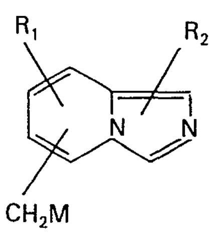

VI

dans laquelle M représente un métal alcalin, chacun des symboles $R_1$ et $R_2$ représente l'hydrogène ou un groupe alkyle en $C_1$-$C_7$, avec un dérivé fonctionnel réactif d'un composé de formule VII

HO-A-B′ (VII)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, ce qui donne un composé de formule Ia

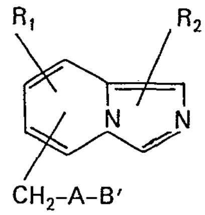

Ia

dans laquelle B′ est différent de B, qu'on convertit en le composé de formule I,

b) ou bien on fait réagir un composé de formule VIII

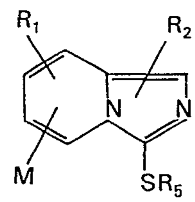

VIII

dans laquelle M représente un métal alcalin, chacun des symboles $R_1$ et $R_2$ représente l'hydrogène ou un groupe alkyle en $C_1$-$C_7$, et $R_5$ représente un groupe alkyle en $C_1$-$C_7$, avec un dérivé fonctionnel réactif d'un composé de formule IX

$HOCH_2$-A-B′ (IX)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, ce qui donne un composé dans lequel B′ est différent de B dans lequel on convertit le groupe B′ en B, et on soumet le composé obtenu à désulfuration,

c) ou bien on fait réagir par catalyse basique un composé de formule générale X

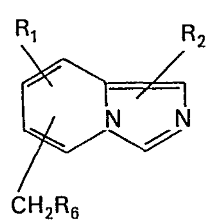

X

dans laquelle chacun des symboles $R_1$ et $R_2$ représente l'hydrogène ou un groupe alkyle en $C_1$-$C_7$, $R_6$ représente un groupe (alcoxy en $C_1$-$C_7$)-carbonyle ou cyano, avec un dérivé fonctionnel réactif d'un composé de formule VII

HO-A-B′ (VII)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, on élimine le groupe $R_6$, ce qui donne un

composé dans lequel B′ est différent de B qu'on convertit en un composé de formule I,

d) ou bien on cyclise un composé de formule XI

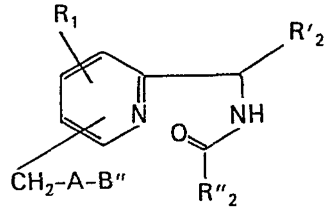

XI

dans laquelle chacun des symboles $R_1$, $R_2'$ et $R_2''$ représente l'hydrogène ou un groupe alkyle en $C_1$-$C_7$, A a les significations indiquées ci-dessus et B″ représente un groupe carboxy, (alcoxy en $C_1$-$C_7$)-carbonyle, carbamoyle non substitué, mono- ou di-substitué par des groupes alkyle en $C_1$-$C_7$, cyano, hydroxyméthyle, (alcanoyle en $C_1$-$C_7$)-oxyméthyle, hydroxyméthyle éthérifié ou halogénométhyle, en un composé de formule Ib

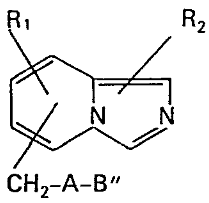

Ib

et on convertit le composé obtenu dans lequel B″ est différent de B en un composé de formule I,

e) ou bien on hydrogène un composé de formule XIV

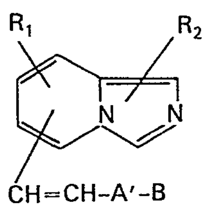

XIV

dans laquelle A′ représente un reste alkylène, alcénylène ou alcynylène à 11 atomes de carbone au maximum,

f) ou bien dans un composé de formule XVI

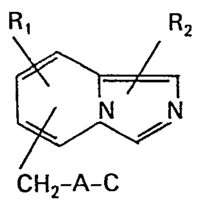

XVI

dans laquelle $R_1$, $R_2$ et A ont les significations indiquées ci-dessus et C représente un reste convertible en un groupe carboxy, on convertit le groupe C, éventuellement avec allongement de la chaîne A dans le cadre de sa définition, en le groupe carboxy,

et, si on le désire, on convertit un composé obtenu répondant à la formule I en un autre composé de l'invention,

et/ou, si on le désire, on convertit un composé obtenu à l'état libre en un sel ou un sel obtenu en le composé libre ou en un autre sel,

et/ou, si on le désire, on sépare un mélange d'isomères ou de racémates obtenus en les isomères ou racémates individuels,

et/ou, si on le désire, on résout des racémates obtenus en les antipodes optiques.

16. Les composés qu'on peut obtenir par le procédé de la rev. 15.

**Revendications pour l'Etat Contractant: AT**

1. Procédé de préparation de nouvelles imidazo[1,5-a]pyridines de formule générale I

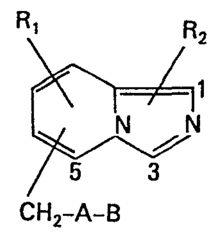

I

ou de leurs dérivés 5,6,7,8-tétrahydrogénés, dans lesquels chacun des symboles $R_1$ et $R_2$ représente l'hydrogène, un halogène ou un groupe alkyle en $C_1$-$C_7$, A représente un groupe alkylène en $C_1$-$C_{12}$, alcynylène ou alcénylène en $C_2$-$C_{12}$, B représente un groupe carboxy, (alcoxy en $C_1$-$C_7$)-carbonyle, carbamoyle non substitué, mono- ou di-substitué par des groupes alkyle en $C_1$-$C_7$, cyano ou hydroxyméthyle, et de leurs sels, caractérisé en ce que:

a) ou bien on fait réagir un composé de formule VI

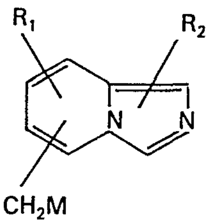

VI

dans laquelle M représente un métal alcalin, chacun des symboles $R_1$ et $R_2$ représente l'hydrogène ou un groupe alkyle en $C_1$-$C_7$, avec un dérivé fonctionnel réactif d'un composé de formule VII

HO-A-B′ (VII)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, ce qui donne un composé de formule Ia

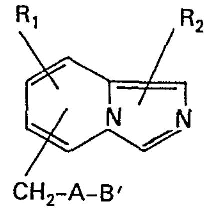

Ia

dans laquelle B′ est différent de B, qu'on convertit en le composé de formule I,

b) ou bien on fait réagir un composé de formule VIII

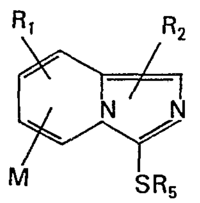

VIII

dans laquelle M représente un métal alcalin, chacun des symboles $R_1$ et $R_2$ représente l'hydrogène ou un groupe alkyle en $C_1$-$C_7$, et $R_5$ représente un groupe alkyle en $C_1$-$C_7$, avec un dérivé fonctionnel réactif d'un composé de formule IX

$HOCH_2$-A-B′ (IX)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, ce qui donne un composé dans lequel B′ est différent de B, dans lequel on convertit le groupe B′ en B, et on soumet le composé obtenu à désulfuration,

c) ou bien on fait réagir par catalyse basique un composé de formule générale X

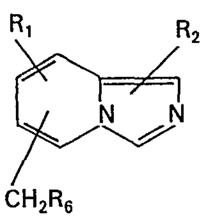

X

dans laquelle chacun des symboles $R_1$ et $R_2$ représente l'hydrogène ou un groupe alkyle en $C_1$-$C_7$, $R_6$ représente un groupe (alcoxy en $C_1$-$C_7$)-carbonyle ou cyano, avec un dérivé fonctionnel réactif d'un composé de formule VII

HO-A-B′ (VII)

dans laquelle A a les significations indiquées ci-dessus, B′ représente un groupe carboxy, trialcoxyméthyle, carbamoyle non substitué, mono- ou di-substitué par des groupes alkyle en $C_1$-$C_7$, cyano, hydroxyméthyle éthérifié ou halogénométhyle, on élimine le groupe $R_6$, ce qui donne un composé dans lequel B′ est différent de B qu'on convertit en un composé de formule I,

d) ou bien on cyclise un composé de formule XI

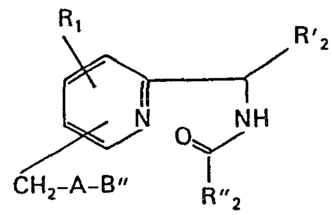

XI

dans laquelle chacun des symboles $R_1$, $R_2'$ et $R_2''$ représente l'hydrogène ou un groupe alkyle en $C_1$-$C_7$, A a les significations indiquées ci-dessus et B″ représente un groupe carboxy, (alcoxy en $C_1$-$C_7$)-carbonyle, carbamoyle non substitué, mono- ou di-substitué par des groupes alkyle en $C_1$-$C_7$, cyano, hydroxyméthyle, (alcanoyle en $C_1$-$C_7$)-oxyméthyle, hydroxyméthyle éthérifié ou halogénométhyle, en un composé de formule Ib

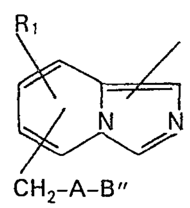

Ib

et on convertit le composé obtenu dans lequel B″ est différent de B en un composé de formule I,

e) ou bien on hydrogène un composé de formule XIV

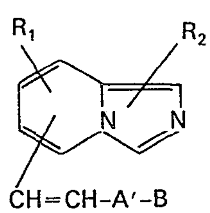

XIV

dans laquelle A′ représente un reste alkylène, alcénylène ou alcynylène à 11 atomes de carbone au maximum,

f) ou bien dans un composé de formule XVI

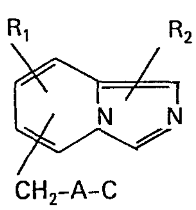

XVI

dans laquelle $R_1$, $R_2$ et A ont les significations indiquées ci-dessus et C représente un reste convertible en un groupe carboxy, on convertit le groupe C, éventuellement avec allongement de la chaîne A dans le cadre de sa définition, en le groupe carboxy,

et, si on le désire, on convertit un composé obtenu répondant à la formule I en un autre composé de l'invention,

et/ou, si on le désire, on convertit un composé obtenu à l'état libre en un sel ou un sel obtenu en le composé libre ou en un autre sel,

et/ou, si on le désire, on sépare un mélange d'isomères ou de racémates obtenus en les isomères ou racémates individuels,

et/ou, si on le désire, on résout des racémates obtenus en les antipodes optiques.

2. Procédé selon la rev. 1, caractérisé en ce que l'on prépare des composés de formule I de la rev. 1 dans lesquels le groupe $CH_2$-A-B est fixé en position 5, et leurs sels.

3. Procédé selon la rev. 1, caractérisé en ce que l'on prépare des composés de formule générale II

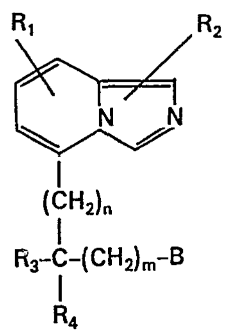

II

ou leurs dérivés 5,6,7,8-tétrahydrogénés dans lesquels chacun des symboles $R_1$, $R_2$, $R_3$ et $R_4$ représente l'hydrogène ou des groupes alkyle en $C_1$-$C_4$, n est un nombre entier de 1 à 7, m est égal à 0 ou 1, B représente un groupe carboxy, (alcoxy en $C_1$-$C_7$)-carbonyle, carbamoyle non substitué, mono- ou di-substitué par des groupes alkyle en $C_1$-$C_7$, cyano ou hydroxyméthyle, et leurs sels.

4. Procédé selon la rev. 1, caractérisé en ce que l'on prépare des composés de formule III

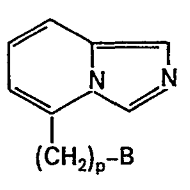

III

ou leurs dérivés 5,6,7,8-tétrahydrogénés dans lesquels p est un nombre entier de 3 à 8, B représente un groupe carboxy, (alcoxy en $C_1$-$C_7$)-carbonyle, carbamoyle non substitué, mono- ou di-substitué par des groupes alkyle en $C_1$-$C_7$, cyano ou hydroxyméthyle, et leurs sels.

5. Procédé selon la rev. 1, caractérisé en ce que l'on prépare des composés de formule IV

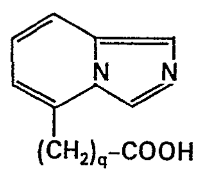

IV

ou leurs dérivés 5,6,7,8-tétrahydrogénés dans lesquels q est égal à 4, 5 ou 6, et leurs sels.

6. Procédé selon la rev. 1, caractérisé en ce que l'on prépare la 5-(5-carboxypentyl)-imidazo-[1,5-a]-pyridine et ses sels.

7. Procédé selon la rev. 1, caractérisé en ce que l'on prépare la 5-(6-carboxyhexyl)-imidazo-[1,5-a]-pyridine et ses sels.

8. Procédé selon la rev. 1, caractérisé en ce que l'on prépare la 5-(6-carboxyhexyl)-5,6,7,8-tétrahydro-imidazo[1,5-a]-pyridine et ses sels.

9. Procédé selon la rev. 1, caractérisé en ce que l'on prépare la 5-(7-carboxyheptyl)-imidazo-[1,5-a]-pyridine et ses sels.

10. Procédé selon la rev. 1, caractérisé en ce que l'on prépare la 5-(5-carboxy-4,4-diméthylpentyl)-imidazo [1,5-a]-pyridine et ses sels.

11. Procédé selon la rev. 1, caractérisé en ce que l'on prépare les composés des rev. 1 à 10 et leurs sels selon les variantes opératoires a) à c) mais sans utiliser les composés de formules VII et IX dans lesquelles B' représente un groupe cyano,

et, si on le désire, on convertit un composé de l'invention ainsi obtenu en un autre composé de l'invention,

et/ou, si on le désire, on convertit un composé obtenu à l'état libre en un sel ou un sel obtenu en le composé libre ou en un autre sel,

et/ou, si on le désire, on sépare un mélange d'isomères ou de racémates obtenu en les isomères ou racémates individuels,

et/ou, si on le désire, on résout les racémates obtenus en les antipodes optiques.

12. Procédé de préparation d'une composition pharmaceutique, caractérisé en ce que l'on combine une substance active selon l'invention, selon l'une des rev. 1 à 10 avec un véhicule pharmaceutique.

13. Procédé de préparation d'une composition pharmaceutique, caractérisé en ce que l'on combine une substance active selon l'invention, selon la rev. 11 avec un véhicule pharmaceutique.